(19) Europäisches Patentamt
European Patent Office
Office européen des brevets

(11) Publication number: **0 142 810 B1**

# (12) EUROPEAN PATENT SPECIFICATION

(45) Date of publication of patent specification: **16.02.94**

(51) Int. Cl.[5]: **G01N 33/545**, G01N 33/531, G01N 33/537

(21) Application number: **84113654.2**

(22) Date of filing: **12.11.84**

## (54) Polymerizable compounds integrally containing antibodies and their uses in polymerization induced separation immunoassays.

(30) Priority: **10.11.83 US 550929**
**16.04.84 US 600838**
**27.01.84 US 574558**
**07.11.84 US 668247**
**07.11.84 US 668248**

(43) Date of publication of application:
**29.05.85 Bulletin 85/22**

(45) Publication of the grant of the patent:
**16.02.94 Bulletin 94/07**

(84) Designated Contracting States:
**AT BE CH DE FR GB IT LI LU NL SE**

(56) References cited:
**FR-A- 2 476 125**
**GB-A- 2 071 669**
**US-A- 3 969 287**
**US-A- 4 195 129**
**GB-A- 1 411 386**
**US-A- 2 853 457**
**US-A- 4 061 466**

**CHEMICAL ABSTRACTS OF JAPAN, vol. 102, 11 February 1985, Columbus, OH (US); p. 311, no. 58887k&NUM;**

(73) Proprietor: **GENETIC SYSTEMS CORPORATION**
**3005 First Avenue**
**Seattle Washington 98121(US)**

(72) Inventor: **Nowinski, Robert C.**
**19520 - 22nd Avenue N. W.**
**Seattle, WA 98177(US)**
Inventor: **Hoffman, Allan S.**
**4528 W. Laurel Drive N. E.**
**Seattle, WA 98105(US)**

(74) Representative: **Patentanwälte Grünecker, Kinkeldey, Stockmair & Partner**
**Maximilianstrasse 58**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

### Technical Field

The present invention relates generally to the de novo synthesis of polymers containing antibodies as an integral part of their structure from conjugation of polymerizable compounds covalently bonded to an antibody, to the use of these conjugates in effecting selective removal of substances from a solution thereof by polymerization of the conjugates and to immunoassays in which a polymerization reaction is used to effect a separation of the specific reactants.

### Background Art

1. Polymer Chemistry

Water-insoluble polymers (such as polysaccharides and polyacrylics) have been commonly used in the fields of biochemistry and immunology (affinity chromotography and immunoassays), as solid-phase supports with passively absorbed or covalently linked antibodies. Such water insoluble polymers are formed by polymerization of compounds, such as monomers or oligomers, to form larger polymeric molecular structures (polymers), or the initiation of copolymerization of monomers with polymerizable polyunsaturated compounds. Polymers may be formed from a single monomeric species (homopolymers), from a mixture of different monomers (copolymers), from polymerizable polyunsaturated compounds containing olefinic or acetylenic unsaturation, or from a mixture of polymerizable polyunsaturated compounds, and one or more monomers.

Linear, branched, or cross-linked structures are possible. By varying the chemical composition or ratios of the monomers, it is possible to form either soluble or insoluble polymers which comprise a broad range of chemical and physical structures. For example, water-soluble monomers (such as acrylamide) can be copolymerized to form water-soluble homopolymers. They can also be copolymerized with less water-soluble monomers (such as N-alkyl or N, N-dialkyl acrylamides) or with cross-linking monomers (such as N, N'-methylenebisacrylamide) to form water-insoluble copolymer structures. Some water-soluble monomers (such as hydroxyethyl methacrylate or acrylonitrile) can be homopolymerized to form water-insoluble homopolymers. See, for example, U.S. Patents 3,957,741; 4,257,884; 4,195,129; 4,225,784; 4,181,636; 4,401,765; and 4,166,105.

To date, the documented coupling of a polypeptide to a polymer has generally occurred under circumstances in which the polypeptide was provided in soluble form and the polymer was provided as a preformed soluble or preformed insoluble material. While these polymers are of utility in providing a surface upon which selective biochemical or immunological reactions can occur, the polymers are of limited value in that the spacing, steric accessibility, and number of polypeptides bound per unit length of polymer cannot be precisely or reproducibly controlled. Lot-to-lot variation is commonly encountered during the manufacture of such solid phase polymer/reactant matrices. In certain end-use applications where reproducibility and standarization are essential (e.g. immunoassays), this variation in composition of the solid-phase polymer/reactant matrices presents a critical problem. Consequently, there is a need in the art for a method to specifically tailor or molecularly engineer polymer compounds incorporating controlled quantities of reactants.

In GB-A-1 411 386 a process is disclosed for the preparation of an immobilised enzyme composite, which process comprises the steps of reacting a hydroxy-containing ethylenically unsaturated monomer with a cyanogen halide; reacting the monomer-cyanogen halide with an aqueous solution of enzyme to form an adduct; and polymerising the monomer-enzyme adduct.

In US-A-2 853 457 polymeric hydrosols composed of an unsaturated acid derivative of a protein and a combination of unsaturated monomers are disclosed. The unsaturated acid derivatives include maleyl, acrylyl and methacrylyl proteins.

2. Immunoassays

Immunoassays have found widespread application in the field of clinical diagnostics for the detection and measurement of drugs, vitamins, hormones, proteins, metabolites, microorganisms, and other substances of interest (analytes) in biological and nonbiological fluids. Typically, these analytes occur in micromolar ($16^{-6}$ M) or less concentration.

Immunoassays generally incorporate antibodies and antigens as reactants, at least one of which is labeled with a signal producing compound (e.g. radioisotope, fluorophore). Following mixture with the sample and incubation, specific antibody/antigen reactions occur (specific binding). The reaction mixture is subsequently interrogated to detect free and specifically-bound labeled reactant, enabling a measurement of the analyte in the sample.

Immunoassays can be divided into two general categories, homogeneous and heterogenous. In a homogeneous immunoassay, the signal emitted by the specifically-bound labeled reactant is different from the signal emitted by the free labeled reactant. Hence, bound and free can be distinguished without physical separation.

The archetypal homogeneous immunoassay is the enzyme-multiplied immunoassay technique (EMIT), which is disclosed in U.S. Patent 3,817,837. In this technology, analyte present in patient sample and analyte/enzyme conjugate compete for a limited amount of anti-analyte antibody. Specific binding of antibody to the conjugate modulates its enzymatic activity. Hence, the amount of enzyme activity is proportional to the amount of analyte in the sample. Homogeneous immunoassays have the advantage of being rapid, easy to perform, and readily amenable to automation. Their principal disadvantages are that they are relatively prone to interferences, are generally limited to low molecular weight analytes and are generally limited in sensitivity to approximately $10^{-9}$ M.

In a heterogeneous immunoassay, the signal emitted by the bound labeled reactant is indistinguishable from the signal emitted by the free labeled reactant. Therefore, a separation step is required to distinguish between the two.

Typical heterogeneous immunoassays include the radioiminunoassay (RIA) and the enzyme-linked immunosorbent assay (ELISA). In the RIA, radiolabeled analyte and analyte present in patient sample compete for a limited amount of immobilized (solid phase) anti-analyte antibody. The solid phase is washed to remove unbound labeled analyte and either the bound or the free fraction is analyzed for the presence of labeled reactant. ELISA assays are performed analogously. In this case, though, the signal is an enzyme instead of a radioisotope. Heterogeneous immunoassays typically employ at least one reagent immobilized on a solid phase. Since the kinetics of reaction between an immobilized antibody (or antigen) and its binding site tend to be slower than the kinetics of the same reaction occurring in solution, long incubation times are frequently required. When the multiple wash steps often needed are considered, it can be appreciated that heterogeneous assays tend to be time-consuming and labor-intensive. However, they are in general more sensitive than homogeneous assays and less prone to interferences, since interfering substances can be removed in the wash steps.

Solids used to immobilize reactants in immunoassays have included controlled pore glass and preformed polymers such as polyvinyls, polyacrylamides, polydextrans and polystyrene.

Numerous separation methods are known in the art and have been used in heterogeneous immunoassays. These include centrifugation, filtration, affinity chromatography, gel permeation chromatography, etc.

The homogeneous immunoassay methods of the prior art are generally prone to interferences, are of limited sensitivity and have a limited range of antigen sizes. The heterogeneous immunoassays of the prior art, while increasing the sensitivity and minimizing interferences, tend to be time consuming and labor intensive. These difficulties generally arise from the added step of physical separation and the need for numerous washes to decrease background interference.

There is a need in the art for an immunoassay method which is sensitive to sub-micromolar concentrations of analyte; which has fast reaction kinetics; and which minimizes the number of manipulations necessary to achieve a result.

## Disclosure of The Invention

One aspect of the invention is a method for the de novo preparation of polymers which integrally contain antibodies as part of their structure from conjugates of polymerizable compounds covalently bonded to an antibody. Polymerizable compounds include monomers, polyunsaturated oligomers, or mixture thereof covalently bonded directly to the antibody or bonded indirectly via an intermediate chemical "spacer arm" compound.

Another aspect of the invention relates to the selective removal of substances from solution by virtue of the ability of the substance to bind to antibodies which are a part of the conjugate. Following specific binding the conjugates are polymerized or copolymerized to remove the specifically bound substances from solution.

Another aspect of the invention relates to bonding of polymerizable compounds to a solid whose surface is modified with the polymerizable compounds so that the polymerizable compounds can

copolymerize with a polymerizable compound/antibody conjugate.

Another aspect of the invention relates to bonding of polymerization initiation compounds to a solid surface such that the surface acts as an initiator during polymerization or copolymerization of the conjugate.

The present invention also provides an immunoassay method for determining the presence of an antigen or hapten in a fluid sample suspected of containing an antigen or hapten, comprising: (a) contacting the fluid sample containing the antigen or hapten with a conjugate of a monomer and an antibody in order to form a polymerizable monomer/antibody-antigen or hapten complex and providing a reporter for labelling the polymerizable monomer/antibody-antigen or hapten complex; (b) separating the labelled complex by initiating the polymerization of the complex; and (c) detecting the incorporation of reporter into the polymerized complex.

Another aspect of the invention provides immunoassay methods for simultaneously measuring two or more antigens or haptens in a fluid sample.

Another aspect of the invention provides immunoassay methods utilizing monomer/antigen or hapten conjugates for competitive immunoassays.

A further aspect of the invention provides monomer/antibody and monomer/antigen or hapten conjugates for use in the immunoassays of the present invention.

A novel feature of this immunoassay is the use of reactant (antibody or antigen) that is covalently linked with a polymerizable organic monomer. Following mixture and reaction of the immunoassay components, the monomer/reactant conjugate and its specific binding complement (i.e., its appropriate antigen or antibody counterpart bound through specific antibody/antigen interactions) can be rapidly and conveniently separated from solution by initiating a polymerization reaction. In contrast to the monomer/reactant conjugate and its specifically bound complement, other components of the immunoassay remain in free solution. Thus, this method provides an effective single-step separation of specifically bound and free reactants.

The polymerization-induced separation immunoassays of the instant invention offer several advantages over prior art homogeneous and heterogeneous immunoassay methods. Because of the separation achieved by polymerization of the monomer/antibody conjugate, the immunoassay of this invention can achieve the sensitivity typical of state-of-the-art heterogeneous techniques combined with the ease of performance of homogeneous techniques.

The immunoassays of this invention can typically be performed in less time than traditional heterogeneous assays because binding reactions which would normally occur on a solid phase can be made to occur in solution instead. Also, the need for extensive washing of the solid phase can be eliminated.

Sandwich immunoassays typically require elution of the specifically bound labeled reactant from the solid phase prior to measurement. This adds an extra step and makes even longer and more cumbersome an already long and tedious process.

The immunoassay of this invention can be performed without the elution of the bound labeled reactant from the polymer, thus simplifying performance.

## Brief Description of the Drawings

Figure 1 is a diagrammatic representation of the synthesis of an activated acrylic acid monomer and a conjugation involving this monomer and, for example, amino groups on an antibody;

Figure 2 is a diagrammatic representation of a polyacrylamide isoelectric focusing gel of the heavy chain of antibody reactant 2HI before and after conjugation with acrylic acid.

Figure 3 depicts the effect of monomer 2-hydroxyethyl methacrylate (HEMA) concentration on the rate of formation of insoluble HEMA homopolymer particles;

Figure 4 depicts the incorporation of monomer/fluorescein tagged antibody reactant conjugates into reactant-containing polymer particles;

Figure 5 is a diagram depicting the relationship between the fluorescence intensity and analyte concentration in one embodiment of the present invention; and

Figure 6 depicts the incorporation of fluoresceintagged monomer/polypeptide conjugates into insoluble polypeptide-containing polymer particles.

Best Mode for Carrying Out the Invention

A. General

One can molecularly engineer polymeric compounds incorporating controlled quantities of antibody for specific applications by first forming a conjugate of a polymerizable compound covalently bonded to an antibody, then polymerizing the conjugate with itself or copolymerizing the conjugate with predetermined amounts of nonderivatized polyunsaturated compounds to form polymers integrally containing the antibody. The conjugates are prepared by covalently linking an appropriate polymerizable compound directly to an antibody or indirectly linking the antibody to the polymerizable compound via an intermediate chemical compound functioning as a spacer arm, then polymerizing the conjugate with itself, or copolymerizing the conjugate with predetermined amounts of polymerizable unsaturated compounds, and/or with a polymerizable compound attached to a solid surface, to form polymers containing the antibody. These conjugates can be used for various applications, such as polymerization-induced separation immunoassays.

For purposes of the present invention the following terms are defined:

Polypeptide - naturally occurring or genetically engineered antibodies (monoclonal or polyclonal).

Integrally containing - the antibody is covalently bonded to the polymer as it is being formed, rather than being coupled or bonded to a preformed polymer.

Analyte - an antigen or hapten the presence or amount of which it is desired to determine.

Biological fluids - blood, blood serum, blood plasma, urine, feces, cerebrospinal fluid, saliva, sputum, cell and tissue derived extracts, etc. in which the analyte is suspected of being contained.

Reactants - naturally occurring or synthetic antigens and antibodies, which are capable of recognizing and specifically binding to each other.

Antigen - molecules which themselves may induce antibodies as well as small molecules which are not capable of eliciting antibody production unless they are coupled to a carrier (e.g. haptens).

Epitope - any antigenic determinant.

Monomer - any polymerizable organic compound which is capable of forming end-to-end covalent linkages (i.e. polymerizing) under the appropriate conditions, and includes certain polyunsaturated oligomers.

Reporter - any substance which is capable of producing a detectable signal either alone or in combination with other reagents, such as, e.g., radioisotopes, fluorophores, chromophores, luminescent compounds, etc.

B. Immunoassays

One aspect of the present invention is directed towards an application of the technology of immunoassays, in which the binding partners of the conjugate are antigen and antibody reactants. The technology may be used (1) to measure one analyte by employing a suitable reporter/reactant conjugate or analytes, (2) to measure simultaneously two or more analytes by employing the corresponding number of reporter/reactant conjugates, each reporter being detectably different.

The methods of this invention for the immunoassay of analytes in biological fluids utilize conjugates of reactants with monomers or signal-producing compounds (monomer/reactant or reporter/reactant conjugates). Separation of free from specifically bound reporter/reactant is effected by a polymerization reaction. Detection of polymer-incorporated signal can be accomplished by a variety of methods, including flow microfluorimetry and filtration.

Although the following discussion pertains primarily to the immunoassay of analytes in biological fluids, it will be appreciated that there are numerous disciplines which require the assay of fluid samples for the presence or amount of organic substances.

The immunoassays of the present invention can be performed in any of several configurations. These can include competitive, sandwich and noncompetitive immunoassay configurations. In every case the analyte of interest can be either an antigen or an antibody. In every case, either reactant (i.e. antigen or antibody) can be conjugated to either labeled substance (i.e. monomer or reporter). The various possible configurations in which immunoassays can be performed are reviewed extensively in Enzyme-Immunoassay, E. T. Maggio (Ed.) CRC Press, Boca Raton, Florida (1980) and numerous other publications.

In one configuration, for example, sample suspected of containing analyte is incubated with a monomer/analyte conjugate and a reporter/reactant conjugate. In this case, the reactant is typically an antibody to the analyte. If the analyte is itself an antibody, the reactant can be a second antibody to the first antibody or it can be the antigen to the first antibody. Analyte present in sample and monomer/analyte

conjugate compete for a limited amount of reporter/reactant. Polymerization-induced separation of free from specifically-bound reporter/reactant enables the detection and measurement of analyte initially present in the sample. This configuration is referred to as competitive.

In the competitive configuration, the immunoassays of this invention can be used to measure both monoepitopic compounds (haptens) and multiepitopic compounds. Multiepitopic is meant to include both compounds having more than one unique epitope and compounds having a single, repeated epitope. Maximum sensitivity is generally attained when the reactant is monovalent with respect to the analyte.

In another configuration, the immunoassays of this invention can be performed as sandwich immunoassays. This configuration is appropriate only for multiepitopic analytes. In the forward sandwich configuration, excess monomer/reactant conjugate is incubated with sample suspected of containing analyte. In this case, the reactant is typically an antibody to the analyte. If the analyte is itself an antibody, the reactant in the reporter/reactant conjugate can be either a second antibody to the first antibody or an antigen to the first antibody. Incubation is carried out under conditions in which specific binding is expected to occur. Following polymerization of monomer/reactant, excess reporter/reactant conjugate is added to the immunoassay mixture. Typically, the reactant is an antibody which binds to a different epitope from that to which the monomer/reactant conjugate binds. Again, if the analyte is itself an antibody, the reactant in the reporter/reactant conjugate can be either an antibody to the first antibody or an antigen to the first antibody. After an appropriate incubation to allow specific binding to occur, the presence or amount of reporter/reactant specifically bound to the polymer is determined. The polymer particles can be washed if desired to remove any free reporter/reactant. In general, however, it is thought sufficient to simply dilute the reaction mixture 2 to 100-fold prior to measuring the amount of reporter associated with the polymer particle. Similarly, if desired, the polymer particles can be separated from solution and the reporter associated with them eluted prior to detection or measurement.

The order of addition of reagents can also be reversed, i.e., sample suspected of containing analyte can be incubated with reporter/reactant prior to addition of monomer/reactant conjugate. This configuration is referred to as a reverse sandwich immunoassay. Likewise, sample, reporter/reactant, and monomer/reactant conjugate can be incubated simultaneously rather than sequentially, in which case the immunoassay is referred to as a simultaneous sandwich immunoassay. Of the three possible sandwich configurations, the simultaneous sandwich immunoassay is most preferred because it requires the least number of manipulations. All three configurations, however, offer significant advantages over prior art sandwich immunoassays in that incubation times are shortened and washing steps are eliminated.

In another configuration, the immunoassays of this invention can be performed by incubating patient sample suspected of containing analyte with monomer/reactant conjugate (the reactant being an antibody to the analyte) under conditions where specific binding is expected to occur. Reporter/reactant can be added sequentially or simultaneously but in this case the reactant is an antibody to the monomer/reactant--analyte complex rather than to the analyte. Following polymerization-induced separation of free from specifically-bound reporter/reactant, the presence or amount of reporter/reactant specifically bound to the polymer particles is determined. In this configuration, the first reactant (anti-analyte) can be conjugated to either monomer or reporter. Likewise, the second reactant (anti-first reactant-analyte complex) can be conjugated to either monomer or reporter, whichever was not conjugated to the first reactant. This configuration, which is referred to an noncompetitive, offers the advantage that both reactants can be employed in excess. Thus, the sensitivity of the immunoassay is not strictly limited by the affinity constants of the reactants This configuration is also appropriate for both monoepitopic and multiepitopic analytes.

Where it is of interest to determine the presence of two or more analytes in a sample simultaneously, multiple reporter/reactant conjugates can be employed in any of the above configurations, each reporter producing a detectably different signal from every other reporter. For example, the presence of two viruses in a single sample, such as lymphadenopathy-associated virus (LAV) and hepatitis B virus, can be determined by employing antibodies to each of the viruses, one conjugated with fluorescein and the other with a phycobiliprotein. The simultaneous measurement of two or more analytes in the same sample could also be of interest in therapeutic drug monitoring, where drugs are co-administered, and in the monitoring of serum proteins, such as the immunoglobulins, and hormones, for example, in thyroid function testing.

The immunoassays of this invention utilize a monomer/reactant conjugate. Typically, the reactant is an antibody or an antigen. Where the reactant is an antibody, either monoclonal or polyclonal antibodies can be used. Prior to conjugation, the antibody will in general be at least partially purified by methods known in the art.

C. Polymerizable Compounds

Polymerizable compounds useful for forming the conjugates of the present invention include polymerizable monomers, polymerizable unsaturated compounds and oligomers.

Polymerizable monomers are typically an ethylenically or acetylenically unsaturated compound containing at least one functionality for coupling to the polypeptide or reactant. Functionalities on the reactant can include, for example, covalently bondable functionalities such as hydroxyl, amine, carboxyl, or sulfhydryl. Olefinically unsaturated monomers can be selected from compounds having the general formula:

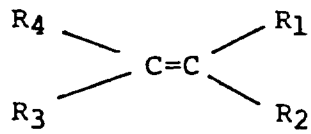

where $R_1$ is H or a lower alkyl radical having from one to eight carbon atoms and $R_2$ can be:
-H
-COCl
-COOH
$-CO_2(CH_2)n\ OH(n = 1\text{-}8)$
$-CH_2NH_2$
$-CH_2Cl$
$-CO_2C_2H_4NHR$ (R = H or any organic group)

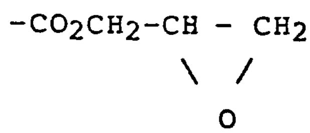

$-CO_2CH_2\text{-}CHOHCH_2OH$
-CHO

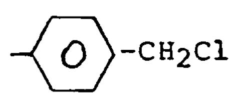

$-CO_2(CH_2)_nNCO\ (n = 1\text{-}8)$

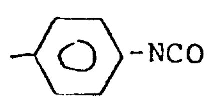

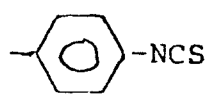

$R_3$ and $R_4$ are most usually H, however they can be chosen to provide an unsaturated group, e.g. any allyl monomer:
$CH_2 = CH\text{-}CH_2\text{-}$
a vinylene monomer:
$-CH = CH\text{-}$
or a diene monomer:

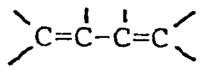

Alternatively, $R_2$ and $R_3$ can be combined as, e.g. -CO-O-CO- to form

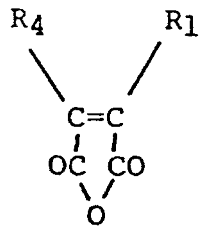

Also, acetylenically unsaturated monomers having a reactable group are also useful:

-C ≡ C-

In addition, polyunsaturated molecules, oligomers or polymers (generally with defined unsaturation) having reactable groups will be useful. For representative examples see: Hoffman, A: S., "Electron Curing of Coatings," Isotopes and Radiation Technology, 9:1, pp. 78-92 (1971).

Polymerizable polyunsaturated compounds which may be used include monomers, oligomers, and polymers containing two or more olefinic or acetylenic groups and at least one reactable site for coupling to a polypeptide, or to an intermediate chemical "spacer" compound. Such polymerizable poly-unsaturated compounds are collectively referred to as monomers for the purposes of this disclosure, although they include certain oligomers or polymers. Oligomers or polymers of the type which may be used include:

Molecules with pendant unsaturation and reactable pendant and terminal groups;

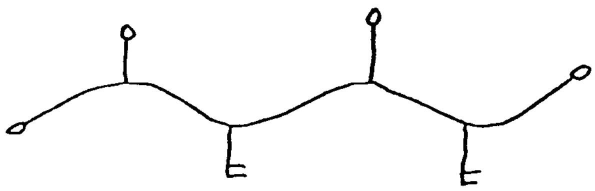

Molecules with pendant and terminal unsaturation and reactable pendant groups;

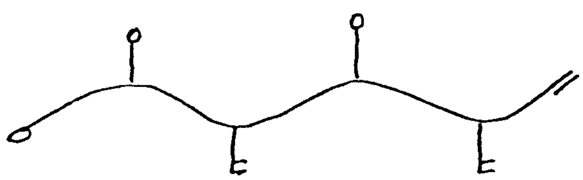

Molecules with both pendant reactable and unsaturated groups;

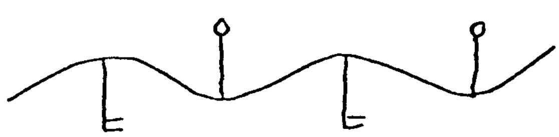

Molecules with pendant unsaturated groups and reactable terminal groups;

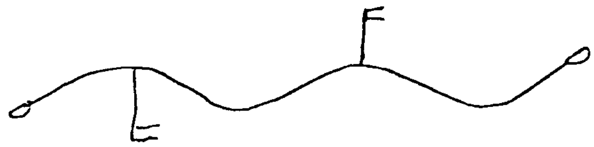

Molecules with pendant unsaturated groups, one unsaturated terminal group and one reactable terminal group;

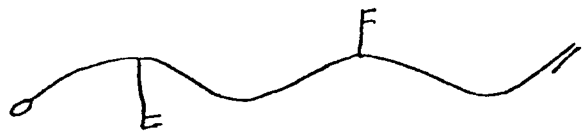

Molecules with backbone unsaturation and reactable terminal groups;

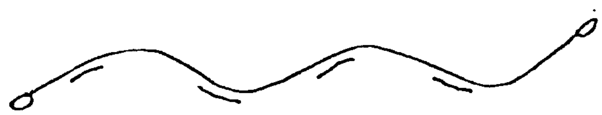

Molecules with backbone unsaturation and one reactable terminal group; and

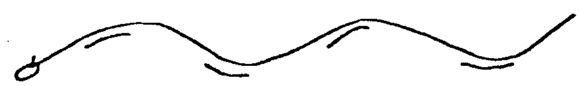

Molecules with one unsaturated terminal group and one reactable terminal group.

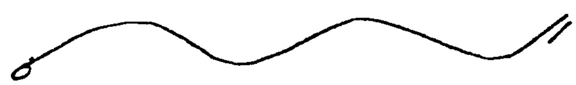

where "--O" indicates a reactive polar group, and " = " indicates unsaturation.

Specific monomers which can be used include acrylic acid, methacrylic acid, acryloyl chloride, methacryloyl chloride, glycidyl acrylate or methacrylate, glycorol acrylate or methacrylate, allylamine, allyl chloride, hydroxylower alkyl-acrylates (e.g., 2-hydroxyethyl methacrylate [HEMA] or 3-hydroxypropyl methacrylate), amino lower alkyl-acrylates (e.g., 2-aminoethylmethacrylate), and vinyl benzoate.

Preferred monomers are those which are soluble in water or water/polar organic solvent mixtures.

Covalent coupling of the polymerizable compound to the reactant or its attached carbohydrate (in the case of glycoprotein reactants) can be carried out by any number of known chemical methods. For example, the polymerizable compound and/or the reactant can be activated to produce a stable but chemically reactive intermediate which can be subsequently reacted. The reactant can also be activated by periodate oxidation of the attached carbohydrates, if the reactant is a glycoprotein. This reaction forms aldehydes which can then condensed with amino groups on the polymerizable compound, such as 2-aminoethyl methacrylate, to form a Schiff base. This Schiff base can be reduced with sodium cyanoborohydride to form a stable covalent linkage. The polymerizable compound may be in the form of an acid halide monomer and may also be directly reacted with the reactant in the presence of an acid scavenger to remove acid as it is formed during reaction. Additionally, bifunctional or hetero-bifunctional reagents may be used. Such bifunctional or hetero-bifunctional reagents are known. In almost all cases, the reaction conditions, i.e., time, temperature, solvent and pH, should be such as to avoid denaturation and/or degradation of the reactant.

A further aspect of the invention relates to chemical bonding of the monomer to a chemical intermediate compounds which functions as a spacer arm, the reactant or polypeptide also being bonded to the chemical

intermediate compound. The spacer arm compound should include reactive sites, for example, covalently bondable functionalities, such as hydroxyl, amine, carboxyl, or sulfhydryl groups. Examples of such compounds include ($\epsilon$-aminocaproic acid, 1,4-diaminobutane, 1,6-diaminohexane, 1,4-butanediol, and p-aminobenzoic acid. Possible advantages of the use of spacers are increased incorporation of the conjugate into the polymer and better reactivity of the reactant portion of the conjugate with its specific binding partner. Both effects are thought to be due to the enhanced accessibility of the conjugate and/or the olefinic or acetylinic groups of the monomer to the bulk solution.

For immunoassay purposes, generally a single species of monomer will be conjugated to a selected reactant or analyte. However, it will be appreciated that a mixture of copolymerizable monomers can be conjugated to the reactant or analyte and which are thereafter separated by polymerization.

Homopolymerization of the conjugate with itself or copolymerization with nonderivatized monomers is initiated by generation of free radicals. Nonderivatized monomers which may be used include, for example, ethylenically and/or acetylenically unsaturated monomers, as previously discussed, alkyl acrylates or methacrylates where the alkyl radical contains from 1 to 8 carbons, acrylonitrile, vinyl acetate and vinyl benzoate. Also, cross-linking compounds may be co-polymerized with the conjugate. Such cross-linking compounds may include, for example N,N'-methylenebisacrylamide or a di-, tri- or tetramethacrylate or acrylate. The percentage of derivatized and nonderivatized monomer may vary from traces up to 100%, but the preferable range is between 0.001 to 100% derivatized monomer and 0 to 99.999% nonderivatized monomer.

The monomer/reactant conjugate, rather than being copolymerized only with nonderivatized polymerizable compounds, may also be reacted with solid surfaces where the surface has been chemically modified to render it reactable with pendant groups attached to a polymerizable compound. Such solid surfaces may be in the form of microbeads, membranes, fibers, porous solids etc. For immunoassay purposes, for example, a polymerizable compound is bonded to a solid surface. The monomer/reactant is incubated with an analyte and a reporter provided for labeling the monomer/reactant conjugate--analyte complex. During copolymerization of the conjugate and the polymerizable compound bonded to the solid surface, the concentration of the reporter at the surface of the solid is enhanced, resulting in a more rapid and sensitive assay. The solid surface may be a polysaccharide, hydrocarbon polymer, fluorocarbon polymer, polyamide, polyurethane, polyester, vinyl polymer, cellulosic polymer or derivative of a cellulosic polymer, glass, silicone polymer.

There are numerous methods by which a polymerizable compound may be attached to a solid polymeric surface.

(a) Solid polymeric surfaces containing aromatic groups such as polystyrene or polyethylene terephthalate, may be treated to render the surface of the solid polymeric surface reactive with pendant groups contained on a monomer. For example, polystyrene microbeads may be reacted with chlorosulfonic acid to yield the corresponding aromatic sulfonyl chlorides which can then be reacted with a pendant amide group attached to a vinyl monomer or with a alkyldiamine to yield a corresponding compound having pendant primary amine groups which are reactive with pendant groups attached to a vinyl monomer having a pendant carboxylic acid conjugate with N-hydroxy succinimide or a pendant acid chloride.

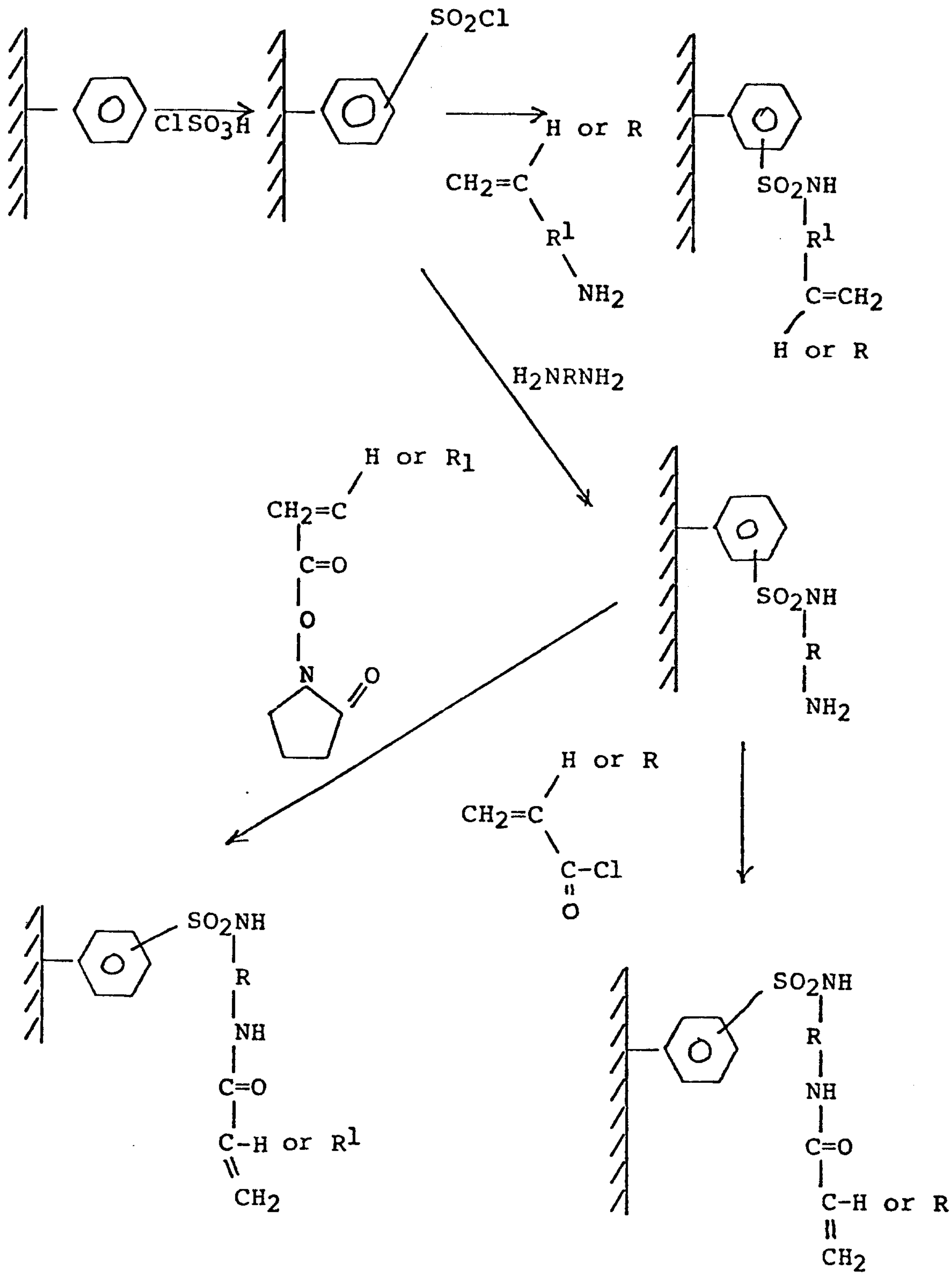


where R and R¹ = alkyl radicals having from 1 to 8 carbon atoms.

(b) Glass surfaces or siliconized surfaces of solids and silicone polymers in whatever form desired i.e., microbead, fiber etc., may be chemically treated to provide surfaces reactive with pendant groups attached to polymerizable compounds, as for example:

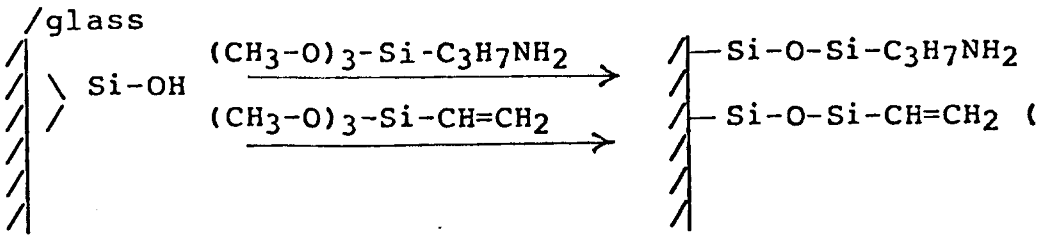


(3)

(4)

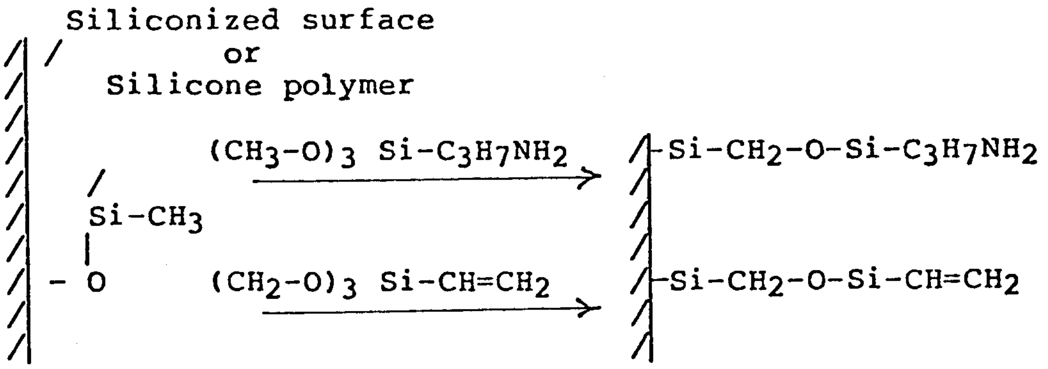


(5)

(6)

React (3) or (5) above with:

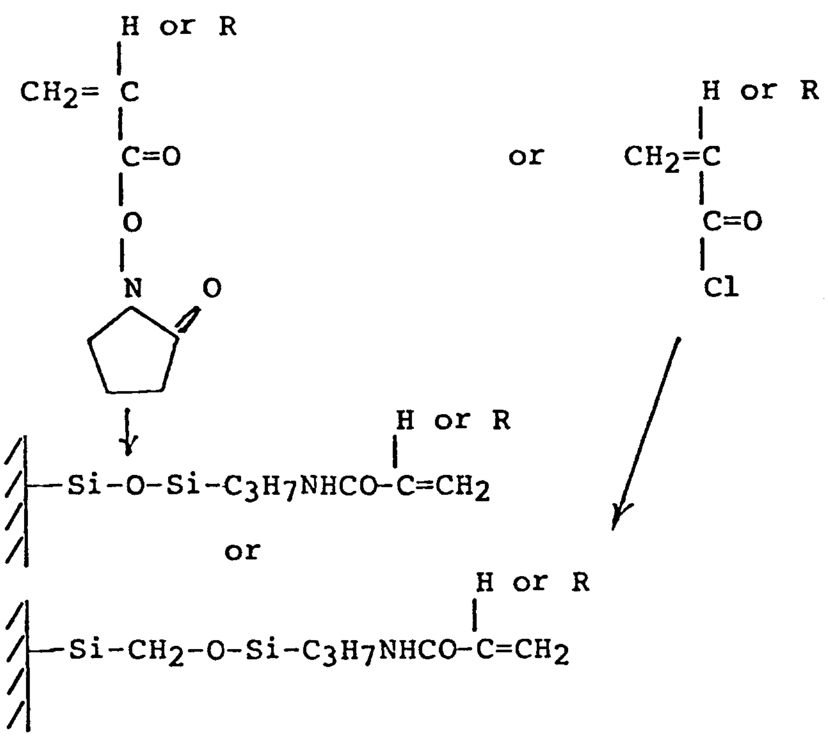

or

(c) Solid polymeric surfaces may be treated with ionizing radiation in the presence of monomers to form a surface having reactive pendant groups such as hydroxyl, carboxyl, amine, or epoxy, where the pendant group can be used for subsequent attachment to a reactive pendant group of a vinyl monomer, as for example:

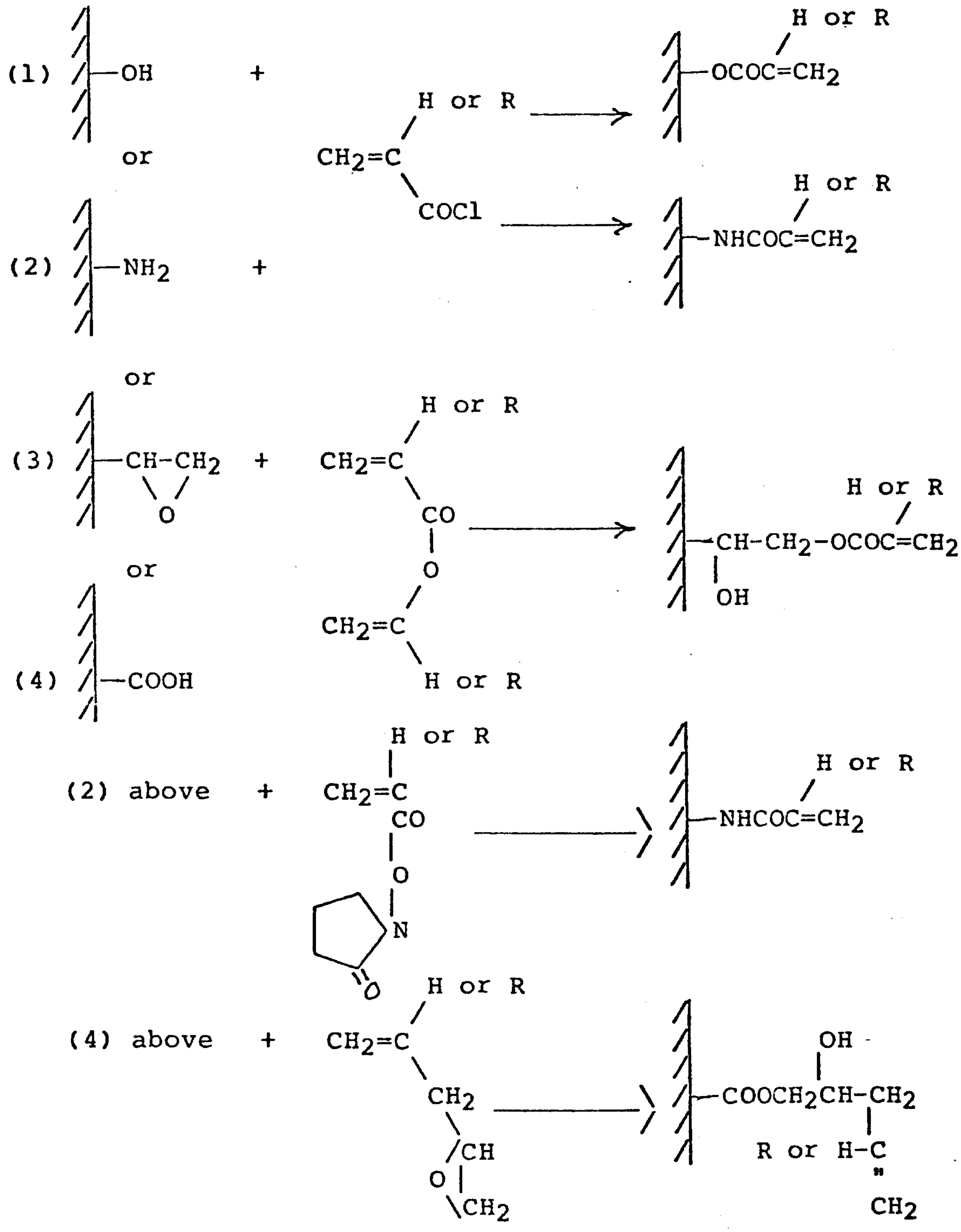

(d) Rather than create reactive sites on polymeric surface, initiation sites for free radical polymerization may be created with oxidizing agents by a number of known methods, including:

1. creation of hydroperoxide and/or peroxide sites on the solid surface by: (a) reaction of the surface with air or oxygen in the presence of ionizing radiation from a radioactive source such as $Co^{60}$ or ultraviolet radiation; (b) subjecting the surface to plasma discharge in the presence of oxygen; or (c) thermally treating the surface in the presence of air, oxygen or ozone. The resulting modified surface is then subjected to heat and/or a reducing agent to form active initiation sites on the solid polymeric surface which are subsequently reacted with vinyl monomer/reactant conjugates and other polymerizable compounds.

2. creation of photosensitive initiation sites on solids or polymeric or protein surfaces by: (a) reacting solid surfaces having pendant reactive groups, such as amine, hydroxyl, sulfhydryl or epoxy groups, to yield a surface which can be reacted with a monomer/reactant conjugate; such as, for example:

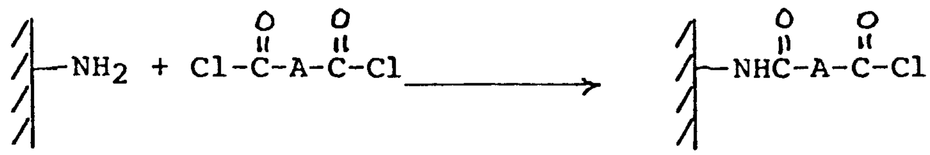

or

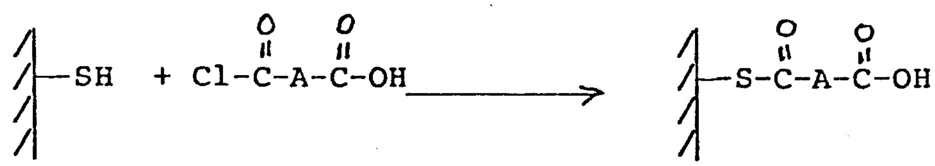

or

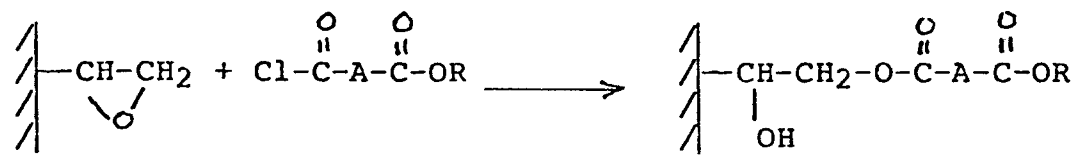

**Where** A =

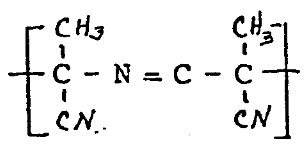

**and** x = 1 to 18

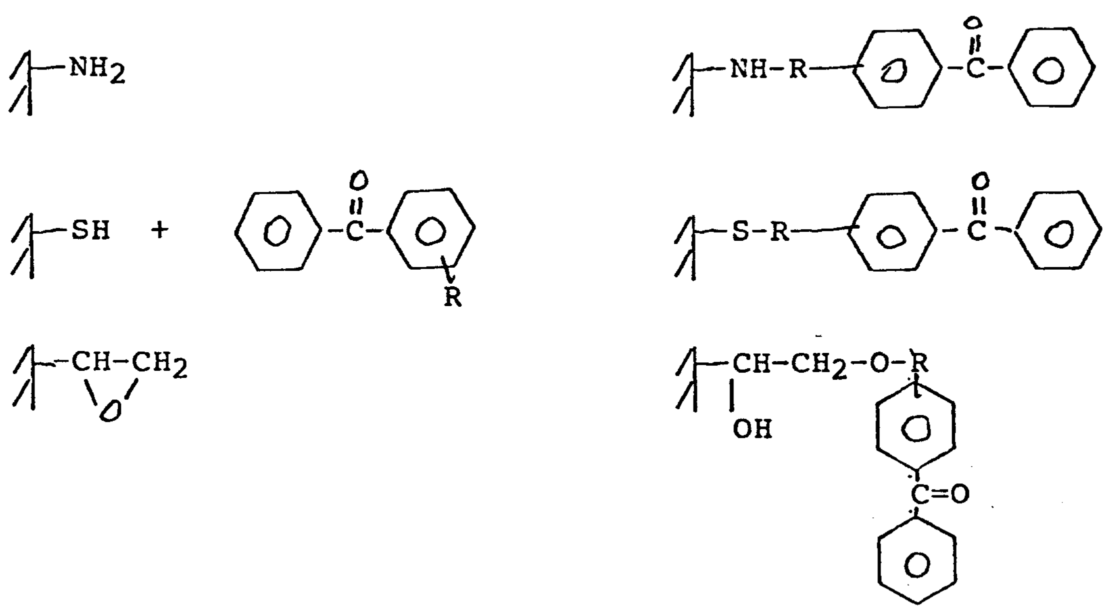

3. forming radiation graft copolymers using monomer incorporating a photosensitizer, as for example,

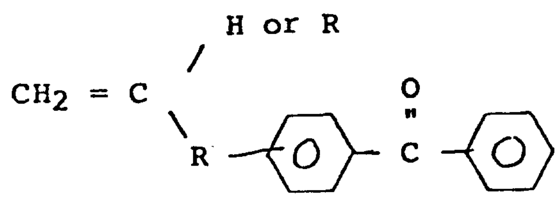

For immunoassay purposes, a monomer/reactant conjugate and a reporter/reactant conjugate are required. The reporter can be chosen from any of those known in the art, including enzymes, fluorophores, radioisotopes, chemiluminescent materials, dye particles, etc. In general, however, fluorophores are preferred. Some suitable fluorophores include fluorescein, rhodamine, phycoerythrin, and Nile blue.

Generally a single species of reporter will be utilized in the present invention. However, it will be appreciated that a mixture of reporter/reactant conjugates can be provided wherein each selected reactant species is conjugated to a distinct reporter. By utilizing an analogous mixture of monomer/reactant conjugates wherein each selected monomer species has a different reactivity, a sample can be assayed for a plurality of analytes simultaneously, as is discussed more fully hereafter. Methods of coupling the reporter to the reactant or analyte are well-known in the art. In general, covalent coupling is preferred, although other means of attachment are possible. The reactive sites which can be utilized for attachment are the same as those discussed above. In general, it is desirable to label the reactant with reporter as heavily as possible without loss of binding activity.

Separation of the specifically bound from the free reactants is accomplished by polymerization of the conjugate. Polymerization or copolymerization with nonderivatized monomer is generally conducted at about room temperature with or without agitation. A surface active agent (e.g. detergent) may or may not be present. Although the reaction may be carried out in the presence of oxygen, it is generally preferred to conduct the reaction in the absence of oxygen or in the presence of a controlled amount of oxygen. The pH range may vary widely from pH 3 to pH 10, although it is preferable to select a pH where the reactant remains the most stable, which is typically between pH 6 and 8. If a surface active agent is used, suitable compounds, such as sodium dodecyl sulfate, sodium stearate, or nonionic materials, such as polyethyleneoxide or lauryl ether, may be employed.

The free radicals may be generated by oxidation-reduction initiation, photochemical initiation, ionizing radiation or thermal initiation. An advantage of both oxidation-reduction initiation and photochemical initiation is production of free radicals at reasonable rates at relatively low temperatures, such as ambient or body temperature (22-37° C). Types of oxidation-reduction initiators which may be used include (1) peroxides in combination with a reducing agent, e.g., hydrogen peroxide with ferrous ion, or benzoyl peroxide with N,N-dialkylaniline or toluidine, and (2) persulfates in combination with a reducing agent, such as N,N,N',N'-tetraethylmethylenediamin (TEMED), sodium metabisulfite or sodium thiosulfate. Specifically, ammonium persulfate, benzoyl peroxide, lauryl peroxide, t-butyl hydroperoxide, t-butyl perbenzoate, cumene hydroperoxide, or mixtures thereof with reducing agents, such as sodium bisulfate or sodium thiosulfate, may be used. It also appears that sodium bisulfite alone may be used for polymerization.

Photoinitiated polymerization may also be used by employing photoinitiators, such as azodiisobutyronitrile, azodiisobutyroamide, benzoin methyl ether, riboflavin, thiazine dyes such as methylene blue and eosin, and transition metals such as ferric chloride or diazidotetramminecobalt (III) azide, in combination with ultraviolet and/or visible light irradiation of the reaction system.

Ionizing radiation may also be employed utilizing radiation from a radioactive source or a particle accelerator.

Polymerization can be carried out in the presence of various physiological materials commonly encountered in biological fluids.

Nonderivatized monomers and polymerization initiating compounds can be present in the reaction mixture throughout the immunoassay or they can be added at any appropriate time. Measurement of the amount of reporter specifically bound to the polymer can be made in any of several ways depending upon the type of signal provided by the reporter. In one embodiment the reporter is a fluorophore and fluorescence associated with polymer particles is detected or measured by flow cytometry and flow microfluorimetry. In another embodiment, fluorescence associated with polymer particles is measured after filtration of the particles.

The following examples are provided by way of illustration, rather than implying any limitation of the present invention.

Experimental

Examples presented here utilize a representative monomer (2-hydroxyethyl methacrylate,[HEMA], two antibodies and an antigen (human IgM) to be detected. The first antibody is a mouse monoclonal designated 2HI, which reacts with the kappa light chain of human IgM and is conjugated with monomer. The second antibody is a mouse monoclonal designated 2C3, which reacts with the mu heavy chain of human IgM and does not interfere with the binding of the first antibody to human IgM. The second antibody is labeled with a fluorescent tag. Briefly stated, these examples utilize a simultaneous sandwich immunoassay configuration in which the presence of the analyte to be detected mediates the incorporation of reporter/reactant (fluorescein labeled 2C3 antibody) into the polymer formed. The amount of reporter/reactant specifically bound to the polymer, i.e. the fluorescence intensity, is proportional to the amount of analyte in the sample. In a typical protocol, the two reactant conjugates are incubated together with the sample suspected of containing analyte to form a ternary complex. Nonderivatized (free) monomer is then added, and subsequent initiation of polymerization results in copolymerization of nonderivatized monomer with monomer/reactant conjugate present in the ternary complex to form a fluorescent polymer. In the absence of analyte, the reporter/reactant conjugate does not form a ternary complex and the polymer particles are not substantially fluorescent.

Example I demonstrates (a) the activation of an acrylic monomer to allow its conjugation to the first reactant, (b) the conjugation of the acrylic monomer to the first reactant including evidence that the conjugation was successful, (c) the demonstration that the monomer/reactant conjugate retained the ability to bind to analyte, and (d) the conjugation of the second reactant with the reporter, fluorescein isothiocyanate. Example II demonstrates the polymerisation of HEMA monomer in a buffered saline solution. In order to assure noninterference in this polymerization process by compounds commonly found in or added to biological samples, this reaction was also conducted in the presence of a sample of serum and in the presence of a nonionic detergent. Example III demonstrates an analytemediated incorporation of fluorescence into the insoluble polymer particles. This was accomplished by first incubating samples of the monomer/reactant conjugate and the reporter/reactant conjugate either with analyte or with a control buffer solution. After subsequent polymerization the particles formed were analyzed by flow cytometry and the analyte positive sample was found to contain highly fluorescent particles, while the particles contained in the control solution exhibited only background fluorescence. Likewise the extent of this fluorescence incorporation into the particles was found to be directly related to the amount of analyte present, forming the basis for a quantative immunoassay system. Example IV demonstrates copolymerization of the monomer/MAb conjugate (MAb/M) with additional nonderivatized HEMA monomer, resulting in synthetic polymer particles that integrally contain MAb in their structure. For the purpose of demonstration, the MAb/M conjugate was first fluorescently tagged with fluorescein isothiocyanate. Polymer particles containing these fluorescein-tagged MAb/M molecules were then visualized under the fluorescence microscope. Additionally, the fluorescence of individual polymer particles was quantitated by flow analysis using a flow cytometer.

Example V illustrates the synthesis of a N-hydroxysuccinimide ester of the monomer vinyl benzoic acid and its conjugation to an antibody reactant. The resultant monomer/antibody conjugate is employed with a fluorescein antibody conjugate (the latter antibody being directed to a second noncompeting epitopic site) in a sandwich immunoassay, wherein the polymer particles formed by copolymerization of the monomer/antibody conjugate with free monomer are separated from the bulk solution by filtration through a 0.45 µm pore size cellulose acetate filter, washed and the signal associated therewith read from the filter using a front surface fluorimeter.

Example VI illustrates a simultaneous two-color immunoassay for IgG and IgM. The same monomer/antibody conjugate is employed as in Example V with two reporter/reactant conjugates, one specific for IgG and conjugated to phycoerythrin and one specific for IgM and conjugated to fluorescein. Measurement of particle-associated red and green fluorescence is made on a flow microfluorimeter.

Example 1

A: Synthesis of an Activated Acrylic Acid Monomer for Conjugation to Reactant.

A mixture containing N-hydroxysuccinimide (NHS) (4.6 g, 40 mmol) and acryloyl chloride (18 mL, 220 mmol) was refluxed with vigorous stirring for 3 hours in an anhydrous atmosphere and the reaction mixture, a homogeneous solution, was evaporated to a syrup. Distilled water (50 mL) was added to the syrup and the mixture was stirred for 30 minutes at 4°C. Upon addition of chloroform (50 mL), the mixture was separated into layers, and the organic layer was extracted successively with water (generally 5 times with 50 mL each time) until the pH of the water layer was approximately 5. The aqueous solutions so obtained were combined and extracted once with chloroform (50 mL); this chloroform solution and the chloroform solution from above were combined, dried over sodium sulfate, and evaporated to a syrup. Crystals, obtained by storing the syrup overnight at -20°C, were triturated with diethyl ether, and harvested by filtration.

Recrystallization from absolute ethanol yielded 2.0 g of the desired product. This compound was analyzed by mass spectrometry, infrared spectroscopy, NMR, liquid chromatography, and melting point, and proved to be the N-hydroxysuccinimide ester of acrylic acid (Figure 1a).

B: Preparation of a Monomer/Reactant Conjugate

The N-hydroxysuccinimide ester of acrylic acid (NSA) was reacted with mouse monoclonal antibody (MAb) 2Hl as follows: 2.2 mg MAb in 0.29 M sodium carbonate buffer, pH 9.3, was added to 20 micrograms of NSA in a total volume of 0.5 mL. The reaction mixture was incubated at 37°C for one hour with constant stirring. Of this solution, 100 microliters was then taken for an analysis by reversed-phase high-performance liquid chromatography (RP-HPLC), which revealed the amount of free acrylic acid (arising from nonspecific hydrolysis of NSA) and remaining NSA in the reaction mix (Table 1)(Figure 1b).

TABLE 1

| RESULTS OF HPLC ANALYSIS OF MONOMER CONJUGATION REACTION MIXTURE | | | |
|---|---|---|---|
| | Antibody | NSA (Activated) monomer) | Acrylic Acid |
| Amount added, nanomoles | 14.5 | 116.0 | 0.0 |
| Amount detected in solution, nanomoles | Not determined | 0.0 | 26.7 |

This indicated that a net of 89 nanomoles of mono mer was attached to the 14.5 nanomoles of MAb for a ratio of 6.2 monomer molecules per MAb.

To remove residual NSA and its hydrolysis products and for further characterization of the derivatized antibody, 200 microliters of the reaction mixture was chromatographed on a column of Sephadex® G-25 (beads of dextran cross-linked with epichlorohydrin from Pharmacia Fine Chemicals AB, Uppsala, Sweden) in the same carbonate buffer to which bovine serum albumin, 0.1 mg/mL, was added to prevent nonspecific adsorption of polypeptides to the Sephadex® G-25.

A sample of the monomer/reactant conjugate was then analyzed by isoelectric focusing. In this procedure, the polypeptide subunits of the proteins were separated according to their isoelectric point, or pH at which they had no net positive or negative charge. For this purpose, the heavy and light chains of the monomer/reactant conjugate were first dissociated in the presence of 3% (w/v) sodium dodecyl sulfate (SDS) and 5% (v/v) 2-mercaptoethanol and separated on the basis of molecular weight by electrophoresis in an SDS-polyacrylamide slab gel. The separated heavy and light chains of the reactant were cut out from the gel and analyzed further by isoelectric focusing in a polyacrylamide slab gel according to their isoelectric point. Staining of the isoelectric focusing gel with dye (Coomassie Brilliant Blue R-250) provided a characteristic pattern of bands for each sample. Since both the heavy and light chains of antibodies are glycoproteins which contain intrinsic variations in their sialic acid content, each heavy and light chain can be separated by a charge into a characteristic family of bands, with each band containing a polypeptide and differing amounts of sialic acid. As the reaction of the activated acrylic acid occurred primarily with amino functional groups on protein lysine residues, the addition of monomer to MAb would be expected to neutralize one positive charge on the protein subunit for each molecule of acrylic acid attached. This in turn would be expected to change the isoelectric point of the derivatized protein.

The results of the isoelectric focusing analysis indicated that each heavy chain was modified by the covalent attachment of approximately three acrylic monomers (Figure 2). Analysis also indicated that the electrophoretic pattern of monomer-derivatized light chain was so close to the nonderivatized polypeptide pattern that essentially minimal conjugation of monomer to light chains had occurred. On this basis, it was estimated that six moles of acrylic acid monomer was conjugated to each mole of antibody (3 per heavy chain times 2 heavy chains per antibody), which was in agreement with the analysis by RP-HPLC.

C: Demonstration that the Monomer/Reactant Conjugate Retained Binding Capacity for Analyte.

To show that the purified monomer/reactant conjugate was still active, it was tested in an enzyme linked immmunosorbent assay (ELISA), and the results indicated no loss of specific binding capacity. For this purpose, human IgG (which contain the same kappa chain antigen as human IgM) was absorbed to the surfaces of wells in a micro ELISA plate (96 wells). The wells were washed, residual nonspecific adsorbing sites on the plastic surface were blocked with bovine serum albumin, and then incubated with serial dilutions of the antibodies (control antibody and monomer/antibody conjugate). The plate was again washed, incubated with goat anti-mouse immunoglobulin conjugated to horseradish peroxidase, o-phenylenediamine and hydrogen peroxide. Dilute aqueous sulfuric acid was added to stop the reaction, the plates were assayed on a micro ELISA reader, and the optical densities of each dilution of monomer/antibody conjugate compared with that of the control antibody. On a molar basis, the monomer/reactant conjugate demonstrated comparable specific binding activity to the nonconjugated antibody alone.

D: Preparation of a Reporter/Reactant Conjugate.

The final step in the assembly of the components of a simultaneous sandwich immunoasssy system was the identification of a second antibody (2C3, which reacts with the mu heavy chain of human IgM) that bound to a different epitope of the analyte, thus it did not block the binding of the first, monomer/reactant conjugate to the analyte. The second antibody reactant was labeled with a reporter (fluorescein). For this purpose, 60 micrograms (20 microliters of a 3.0 mg/mL solution in DMSO) of fluorescein isothiocyanate isomer II (FITC) was added to 1 milligram of antibody 2C3 in 0.125 mL of 0.27 M carbonate buffer, pH 9.3. The mixture was incubated for 30 minutes at 37° C and chromatographed on a column of Sephadex® G-25 in phosphate buffered saline to which 0.5 M NaCl and 0.1% $NaN_3$ had been added. This separated the fluorescein labeled antibody from any free FITC that remained in solution. The peak was collected in a volume of 0.25 ml and the fluorescein-to-antibody ratio, calculated from the absorbences at 280 nm and 495 nm using the equation F/Ab ratio = 3.1 x A495/A280 - 0.31 x. A495, was found to be 4.7. Using methods similar to those in Example I.C, this reporter/reactant was found to be fully capable of specifically binding to analyte.

Example II

POLYMERIZATION OF HEMA MONOMER IN A BUFFERED SALINE SOLUTION

Polymerization of 2-hydroxyethyl methacrylate in the presence of physiological compounds was carried out as follows: to 2.73 mL of distilled water or phosphate-buffered saline, pH 7.4, was added 0.06 to 0.24 mL of 25% (v/v) 2-hydroxyethyl methacrylate (HEMA, Aldrich Chemical Company). Water was added to a final volume of 2.97 mL, as necessary. After bubbling prepurified nitrogen through a Pasteur pipette into the bottom of the cuvette for at least five minutes, 30 microliters of 1 M $Na_2S_2O_5$ was added and the precipitation of the resulting polymer was followed at 550 nm with a Beckman Model 26 spectrophotometer. Figure 3 illustrates the dependence of the rate of precipitation on the concentration of monomer. From this data, a concentration of 2% was chosen.

Inclusion of fetal calf serum, up to 10% (v/v), or "Nonidet®P-40", a nonionic detergent, available from Shell Chemical Co., at concentrations up to 1% (w/v), had no effect on the rate of formation of the polymer particles. Since fetal calf serum contains a variety of proteins and other physiological compounds, this indicates that most proteins and physiological compounds will not inhibit formation of the polymer particles. Since nonionic detergents are commonly used in immunoassays to solubilize biological substances, this indicates that it will be possible to utilize detergents in polymerization-induced separation immunoassays without interference.

Example III

DEMONSTRATION OF ANALYTE-SPECIFIC INCORPORATION OF FLUORESCENCE INTO POLYMER.

This example illustrates a simultaneous two antibody sandwich immunoassay method. In this method, the reporter/reactant (fluorescein-labeled 2C3, 5 micrograms), the analyte (human IgM 4.5 micrograms) and the monomer/reactant (acrylic acid-labeled 2HI, 5 micrograms) were incubated together, which resulted in the formation of a ternary complex or sandwich containing both monomer and reporter. Copolymerization of this complex with additional nonderivatized monomer (HEMA) resulted in the formation of fluorescent, polymer particles (sample a).

For comparison, a control sample was prepared that was identical to the first except the analyte was omitted. This resulted in the formation of polymer particles that contained monomer/reactant but not reporter/reactant, hence the polymer particles were nonfluorescent (sample b). To quantitatively compare the amounts of incorporation of fluorescence into the polymer particles from the two samples, they were subjected to quantitative flow analysis using a flow cytometer.

After the polymerization had proceeded for ten minutes, the suspension of polymer particles was diluted one-hundred-fold and then introduced into a flow cytometer (Becton Dickinson, FACS IV) equipped with an Argon ion laser light source. In this procedure, the suspended particles were carried single-file in a laminar stream of buffer. Interrogation of the particle stream with the laser beam generated light scatter each time a particle entered the laser pathway. The extent of the light scatter was a reflection of particle size and shape. The measurement of light scatter is used to electronically trigger a simultaneous measure of fluorescence emitted from the particle. In this way, fluorescence specifically associated with polymer particles can be selectively measured.

The results are graphically presented in Figure 4, which compares the fluorescence intensity of copolymer particles from a sample a (dotted line) with the fluorescence intensity of copolymer particles from a control sample b (solid line), from which the analyte was omitted. The fluorescence intensity of the copolymer particles formed in the presence of analyte (complete system) was shifted over 73 channels relative to the control (see arrows in Figure 4). The fluroescence intensity scale (x axis) is logarithmic, and a shift of 73 channels corresponded to an 20-fold increase in fluorescence intensity. This increase in fluorescence intensity proved to be a linear function of the amount of analyte present in the sample. Figure 5 is a plot of fluorescence intensity, on a linear scale, against the amount of analyte present in the sample, using 1 microgram of each reactant conjugate and otherwise the same conditions as used in Figure 3.

Example IV

DEMONSTRATION OF INCORPORATION OF MONOMER/POLYPEPTIDE CONJUGATE INTO POLYMER

In order to provide a means of identifying and monitoring the presence of polypeptides in polymers, the monomer/antibody conjugate (MAb-M) was covalently tagged with a fluorescent compound. For this purpose, 88 micrograms (8.8 microliters of 10 mg/mL in DMSO) of fluorescein isothiocyanate isomer II (FITC) was added to 3.6 mg MAb-M in 1.2 mL of 0.29M carbonate buffer, pH 9.3. The mixture was stirred for 1 hour at 37 °C and chromatographed on a column of Sephadex® G-25 in phosphate-buffered saline to which bovine serum albumin (0.01 mg/mL) had been added to prevent nonspecific adsorption to the column. This separated the fluorescein-tagged MAb-M from any free fluorescein isothiocyanate that remained in solution.

The fluorescein-tagged MAb-M conjugates were then copolymerized with additional HEMA to form insoluble polymer particles. Two methods were used to demonstrate the incorporation of fluorescein-tagged MAb-M into the de novo synthesized polymer. Both methods, fluorescence microscopy and quantitative flow analysis with the fluorescence activated cell sorter, actually measured the presence of fluorescence in the polymer particles. For comparison, two controls were used (samples a and b). In sample a, HEMA was polymerized by itself into insoluble polymer particles in a smaller version of the polymerization system of Example I (total volume: 1ml). In sample b, 50 micrograms of a fluorescein-tagged (but not monomer-conjugated) "bystander" MAb was added to the polymerization system to test for nonspecific entrapment of bystander polypeptides during the formation of insoluble polymer particles. In neither case was any fluorescence seen to be associated with the polymer particles when viewed in the fluorescence microscope. In sample c, 50 micrograms of monomer-conjugated, fluorescein-tagged MAb-M was added to the polymerization system to test for specific incorporation of the polypeptide, via copolymerization, into the polymer particles. In this case, all of the fluorescence was seen to be associated with the polymer particles

when viewed in the fluorescence microscope.

The same experiment was examined by quantitative flow analysis with a fluorescence-activated cell sorter. After the polymerization had proceeded for ten minutes, the suspension of polymer particles was diluted one-hundred-fold and then introduced into a flow cytometer (Becton Dickinson, FACS IV) equipped with an Argon ion laser light source. In this procedure, the suspended particles were carried single file in a laminar stream of buffer. Interrogation of the particle stream with the laser beam generated light scatter each time a particle entered the laser pathway. The extent of the light scatter was a reflection of particle size and shape. Further, measurement of light scatter can also be used to electronically trigger a simultaneous measure of fluorescence emitted from the particle which was responsible for the light scatter signal. In this way, fluorescence specifically associated with polymer particles can be selectively measured.

The results can be summarized by reference to Fig. 6 as follows: Light Scatter Analysis. Light scatter analysis (panels A and C) of polymer particles formed from HEMA alone (solid line), polymer particles formed from HEMA polymerized in the presence of bystander antibody (dotted line), the polymer particles formed by copolymerization of HEMA with fluorescein-tagged MAb (dashed line) , shows that the particle size distribution was substantially the same for all three samples. Fluorescence Analysis. Panel B compares the fluorescence intensity of polymer particles formed from HEMA alone (solid line) and polymer particles formed from HEMA polymerized in the presence of bystander antibody (dotted line). The fluorescence intensity was substantially the same for both samples. Since the intensity was substantially the same regardless of whether or not fluorescein-tagged bystander antibody was present, the weak fluorescence signal of both samples was assumed to be due to autofluorescence of the HEMA polymer itself and indicated that there was minimal nonspecific entrapment of the bystander antibody in the polymer. Panel D compares the fluorescence intensity of polymer particles formed from HEMA alone (solid line) and those formed by copolymerization of HEMA with the monomer-derivatized, fluorescein-tagged MAb (dashed line). The fluorescence intensity of the copolymer particles (monomer-derivatized, fluorescein-tagged Mab and HEMA) was shifted over 28 channels. The fluorescence intensity scale (x axis) is logarithmic, and a shift of 28 channels corresponded to a three-fold increase in fluorescence intensity. This dramatic increase in the fluorescence intensity provided conclusive evidence that the monomer-derivatized, fluorescein-tagged MAb was integrally incorporated into the polymer particles.

## Example V

### A: Synthesis of the N-Hydroxysuccinimide (NHS) Ester of 4-Vinyl Benzoic Acid (VBA)

To 1 g (6.74 mmoles) of VBA in 40 ml of tetrahydrofuran (THF) were added 0.775 g of NHS and 1.39 g of dicyclohexyl (carbodiimide). The resultant mixture was stirred overnight at room temperature. The mixture was filtered and a small amount of p-methoxyphenol was added to the filtrate. The filtrate was then evaporated to dryness, yielding pale yellow crystals which were subsequently triturated with diethyl ether. The resultant solid was recrystallized once from methanol. Analysis by NMR, mass spectrometry, and infrared spectroscopy confirmed the identity of the compound as N-succinimidyloxy-(4-vinyl benzoate) (NSB).

### B: Conjugation of NSB to Monoclonal Antibody

A murine monoclonal antibody designated 2HI, which is reactive with kappa light chains of human immunoglobulin, was conjugated to NSB to yield a monomer/antibody conjugate. Briefly, to 7 mg of purified 2HI IgC in 1.23 mL of 0.29 M carbonate buffer (pH 9.3) was added 113 micrograms of NSB in 11.3 microliters of THF. The mixture was stirred for 1 hour at 37 °C and then chromatographed on a Sephadex® G-25 column (PD-10 disposable column from Pharmacia Fine Chemicals, Piscataway, N.J.) which had been equilibrated in phosphate buffered saline (PBS, pH 7.4). Fractions were collected and their absorbance at 280 nm determined. The peak fractions were pooled, divided into 100 microliter aliquots, and stored frozen at -20 °C until needed.

### C: Conjugation of Fluorescein Isothiocyanate (FITC) to Monoclonal Antibody

A murine monoclonal antibody, designated 3F6, which is reactive with gamma heavy chains of human immunoglobulin, was conjugated to FITC to yield a reporter/antibody conjugate. Briefly, 60 micrograms (20 microliters of a 3.0 mg/mL solution in DMSO) of FITC isomer II was added to 1 mg of purified 3F6 IgG in 0.125 mL of 0.29 M carbonate buffer (pH 9.3). The mixture was incubated for one-half hour at 37 °C and

then chromatographed on a column of Sephadex® G-25 equilibrated in PBS/0.5M NaCl/0.1% (w/v) $NaN_3$. Fractions were collected and the fluorescein-to-antibody protein (F/P) ratio of the peak fractions determined using the following equation:

$$F/P = \frac{(3.1 \times A_{495\ nm}) - (0.31 \times A_{495\ nm})}{A_{280\ nm}}$$

The F/P ratio was found to be approximately 5.

D: Polymerization-Induced Separation Immunoassay for Human IgG

An immunoassay for human IgG was performed using NSB-conjugated antibody 2HI ($Ab_M$) and FITC-conjugated 3F6 ($Ab_F$). An IgG/kappa human myeloma protein was used as the antigen.

The assay was performed as follows: $Ab_M$ (30 microgram/mL final concentration), $Ab_F$ (30 micrograms/mL final concentration), and hydroxyethylmethacrylate (HEMA, 1% (w/v) final concentration) were admixed with sample containing antigen in a total volume of 100 microliters. After a ten minute incubation at 37° C, polymerization was initiated by the addition of 25 microliters of 30 mM ammonium persulfate and 25 microliters of 240 mM N,N,N'N'-tetraethylmethylenediamine (TEMED). Polymerization was conducted for twenty minutes at 37° C. The amount of fluorescence incorporated into the resultant copolymer particles was determined either by flow microfluorimetry or by filtration on a Screen Machine (Pandex Laboratories, Mundelein, Ill.). In the flow microfluorimetry method, a 50 microliter aliquot of the reaction mixture was diluted into 2 mL of PBS prior to analysis on a FACS IV (Becton-Dickinson, Sunnyvale, Ca.). In the filtration method, a 50 microliter aliquot of the reaction mixture was diluted with an equal volume of PBS containing 0.05% (w/v) Tween 20® and added to a well of a Screen Machine microtiter tray. The wells in these trays contain 0.45µm (micron) average pore size cellulose acetate filters. The filters were pre-equilibrated by washing with 1% (w/v) BSA in PBS.

Table I compares the relative fluorescence intensity obtained for various concentrations of antigen using the two methods of measurement.

Table I

| Antigen Concentration (micrograms/mL) | Relative Fluorescence Intensity | |
|---|---|---|
| | Flow Microfluorimetry | Filtration ($X10^{-2}$) |
| 0 | 2.9 | 11.7 |
| 0.007 | 4.0 | 12.9 |
| 0.015 | 4.7 | 13.4 |
| 0.03 | 5.8 | 17.5 |
| 0.06 | 9.0 | 25.0 |
| 0.125 | 16.0 | 37.2 |
| 0.250 | 27.2 | 64.0 |

Example VI

Polymerization-Induced Separation Immunoassay for the Simultaneous Two-Color Measurement of Human IgG and IgM

NSB-conjugated 2HI ($Ab_M$, to human kappa light chains) and FITC-conjugated 2C3 ($AB_F$, a murine monoclonal to human mu heavy chains) were prepared as described above.

A: Conjugation of R-Phycoerythrin to Monoclonal Antibody

The murine monoclonal antibody, designated 3F6, which is reactive with gamma heavy chains of human immunoglobulin, was conjugated to R-phycoerythrin (PE) to yield a reporter/antibody conjugate. R-phycoerythrin was purified from the red alga Porphyra yesoensis as described by Oi et al., J. Cell Biol., 93:981, 1982. Conjugation occurred via sulfoether linkage, using succinimidyl 4-(N-maleimidomethyl) cyclohexane-1-carboxylate (SMCC) as a bifunctional cross-linking agent. Briefly, thiol groups were introduced onto the antibody using S-acetylmercaptosuccinic anhydride, while reactive maleimide groups were introduced onto PE using SMCC. The PE derivative was then coupled to the antibody derivative.

The introduction of thiol groups into 3F6 was accomplished as follows: To 2.5 mg of purified 3F6 IgG in 0.5 mL of PBS was added 5 microliters of a 60 mg/mL solution of S-acetylmercaptosuccinic anhydride in DMF. The mixture was incubated at room temperature with continuous stirring for one-half hour, after which 0.5 mL of 1 M hy droxylamine, pH7.0 and 0.05 mL of 0.1 M Tris buffer, pH 7.0, containing 0.2 M EDTA were added.

Incubation was continued for ten minutes at room temperature, after which the reaction mixture was chromatographed on a Sephadex G-25® column (1.0 x 45 cm) equilibrated in 0.02 M phosphate buffer, pH 6.0, containing 0.005 M EDTA. Fractions were collected and their absorbance at 280 nm determined. The peak fractions were pooled and the number of thiol groups per mole of IgG determined according to the method of Grassetti, et al., Arch. Biochem. Biophys. 119:41, 1967. Approximately seven thiols were introduced per mole of IgG.

Reactive maleimide groups were introduced into PE as follows: To 1.0 mg of PE in 1.0 mL of PBS was added 25 microliters of a 0.9 mg/mL solution of SMCC in DMF. The mixture was incubated at room temperature for one-half hour, after which it was chromatographed on a Sephadex G-25® column (1.0 x 45 cm) equilibrated in 0.1 M phosphate buffer, pH 6.5. Fractions were collected and their absorbence at 280 nm monitored. The peak fractions were pooled and the molar ratio of SMCC to PE determined accordingly to the method of Ishikawa, J., Immunoassay 4:209, 1983. Approximately four molecules of SMCC were introduced per molecule of PE.

The resultant PE derivative was coupled to thiolated 3F6 as follows: To 1 mg of PE derivative in 2 mL of 0.1 M phosphate buffer, pH 6.5, was added 1.5 mg of thiolated 3F6 in 1 mL of 0.02 M phosphate buffer, pH 6.0, containing 0.005 M EDTA. After mixing, 0.3 mL of 10X PBS was added and the resultant mixture was incubated at room temperature for two hours. After incubation, 50 microliters of 0.1 M N-ethylmaleimide in DMF was added. Incubation was continued for 10 minutes, after which the reaction mixture was chromatographed on a Sephacryl S-300® column (1.5 x 120 cm) equilibrated in PBS containing 0.1% (w/v) aside. Two PE-containing peaks were obtained, the higher molecular weight peak corresponding to 3F6-conjugated PE and the lower molecular weight peak, to unconjugated PE. The fractions corresponding to the high molecular weight peak were pooled and found to have a molar ratio of 3F6 to PE of 1.5. This conjugate is referred to as $Ab_{PE}$.

B: Simultaneous Two-Color Immunoassay of Human IgG and IgM

A polymerization-induced separation immunoassay for the simultaneous two-color measurement of human IgG and IgM was conducted as follows: $Ab_m$ (30 micrograms/mL final concentration), $Ab_F$ (45 micrograms/mL final concentration), $Ab_{PE}$ (30 micrograms/mL final concentration) were admixed with sample containing varying amounts of both antigens in a total volume of 100 microliters. An IgG/kappa myeloma protein and an IgM/kappa myeloma protein were used as antigens. After a ten-minute incubation at 37 °C, polymerization was initiated by the addition of 25 microliters of 30 mM ammonium persulfate and 25 microliters of 240 mM TEMED. After 20 minutes polymerization, a 50 microliters aliquot of the reaction mixture was diluted into 2 mL of PBS for analysis by multiparameter flow microfluorimetry using a FACS IV. A 488 µm laser line was used for excitation. A 560 µm dichroic mirror (Becton-Dickinson) was used to split the emission wavelengths. Additionally, 580 µm longpass and 540 µm shortpass filters (Ditric Optics, Hudson, Ma.) were placed in front of the red (PE) and green (FITC) photomultiplier tubes, respectively. A compensator was used to correct any residual spillover of green and red signals.

Simultaneous two-color measurement of IgG and IgM was carried out in a series of samples with presentation of the data as number of particles (vertical axis) versus log green fluorescence versus log red fluorescence on a 64 x 64 dot grid with each 4.5 dots representing an approximate doubling of fluorescence. A first fluorescence profile of copolymer particles was formed in the presence of 0 micrograms/mL IgM and 1 microgram/mL IgG and fluoresced, with no green fluorescence above background. A second fluorescence profile of copolymer particles formed in the presence of 1 micrograms/mL

IgM and 0 micrograms/mL IgG particles fluoresced green. A fluorescence profile obtained by mixing the copolymer particles formed in the first profile with those formed in the second profile resulted in two distinct peaks, a green peak which contains the IgM bearing particles and a red peak which contains the IgG-bearing particles.

Fluorescence profiles of copolymer particles formed in the presence of a constant amount of IgG (1 micrograms/mL) and varying amounts of IgM (0 micrograms/mL final concentration, 0.125 micrograms/mL, 0.500 micrograms/mL, and 1.0 micrograms/mL, respectively). The copolymer particles formed emitted both green and red fluorescence and, when graphed, form a single peak which moves out along both axes in proportion to the amount of each antigen present in the sample.

## Claims

**1.** A conjugate used in the de novo preparation of polymers containing one or more antibodies, comprising:
a polymerizable compound containing at least one reactive site for bonding with an antibody, and
an antibody covalently bonded thereto.

**2.** The conjugate of Claim 1 wherein the antibody is selected from the group consisting of naturally occurring monoclonal, polyclonal, chemically synthesized and recombinant DNA (rDNA)-derived antibodies.

**3.** The conjugate of Claim 1 wherein the antibody is bonded to an intermediate "spacer arm" compound which is also bonded to the polymerizable compound.

**4.** The conjugate of Claim 1 wherein the polymerizable compound is soluble in water or water/polar organic solvent mixtures.

**5.** The conjugate of Claim 1 or 3 homopolymerized to form a polymer integrally containing the antibody.

**6.** The conjugate of Claim 1 or 3 copolymerized with nonderivatized monomer to form a copolymer integrally containing the antibody.

**7.** The conjugate of claim 1 or 3 wherein the polymerizable compound is a compound containing olefinic or acetylenic unsaturation and at least one reactive site for bonding with the antibody or intermediate "spacer arm" compound.

**8.** The conjugate of claim 1 or 3 wherein the polymerizable compound is a polyunsaturated oligomer having at least one reactive site for bonding with the antibody or intermediate "spacer arm" compound.

**9.** The conjugate of Claim 1, the polymerizable compound comprising a polymerizable, ethylenically unsaturated organic monomer selected from the group consisting of:

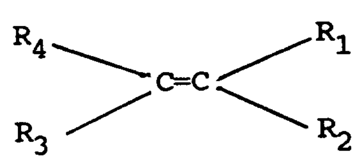

wherein $R_1$ is hydrogen or a lower alkyl having from one to eight carbon atoms and $R_2$ is selected from the group consisting of
-H
-COCl
-COOH
$-CO_2(CH_2)_nOH$ ($n$ = 1-8)
$-CH_2NH_2$
$-CH_2Cl$
$-CO_2C_2H_4NHR$ (R = H or any organic group)

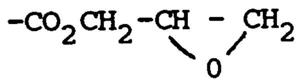

$-CO_2CH_2-CHOHCH_2OH$

-CHO

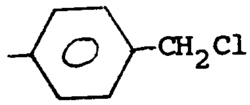

$-CO_2(CH_2)_nNCO$ (n = 1-8)

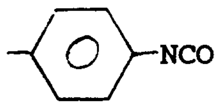

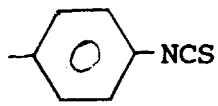

and $R_3$ and $R_4$ are selected from the group consisting of H and/or a residue which will provide a further unsaturated group.

**10.** The conjugate of Claim 9 wherein $R_3$ and $R_4$ are H.

**11.** A method of selectively removing an antigen or hapten of interest from a fluid mixture, comprising:

a) contacting the antigen or hapten in the fluid mixture with a conjugate of a monomer and an antibody that has specific binding activity for said antigen or hapten to bind the monomer to the antigen or hapten to form a polymerizable monomer/antibody-antigen or hapten complex; and

b) polymerizing the complex to form an insoluble polymer which can be separated from the fluid mixture.

**12.** The method of Claim 11 wherein the fluid mixture is aqueous.

**13.** The method of Claim 11, including providing a solid surface having at least one polymerizable compound bonded thereto capable of copolymerizing with the polymerizable conjugate of a monomer and an antibody, and polymerizing the conjugate in the presence of the polymerizable compound bonded to the solid surface.

**14.** An immunoassay method for determining the presence of an antigen or hapten in a fluid sample suspected of containing an antigen or hapten, comprising:

(a) contacting the fluid sample containing the antigen or hapten with a conjugate of a monomer and an antibody in order to form a polymerizable monomer/antibody-antigen or hapten complex and providing a reporter for labelling the polymerizable monomer/antibody-antigen or hapten complex;

(b) separating the labelled complex by initiating the polymerization of the complex; and

(c) detecting the incorporation of reporter into the polymerized complex.

**15.** The immunoassay method of Claim 14 wherein the reporter is provided by a reporter/antibody conjugate capable of recognizing and binding to the antigen or hapten, to the conjugate of a monomer and an antibody or the monomer/antibody-antigen or hapten complex.

**16.** The immunoassay method of Claim 14 wherein more than one antigen or hapten is determined in the fluid sample and

wherein, in step a), for each antigen or hapten to be determined a conjugate of a monomer and an

antibody is added and further a reporter for labeling each of the monomer/antibody-antigen or hapten complexes is provided, each reporter providing a detectably different signal from every other reporter present in said fluid sample, and
wherein in step c) the incorporation of each reporter into each of the previously separated insoluble polymers is detected.

**17.** An immunoassay method for determining the presence of an antigen or hapten in a fluid sample suspected of containing the said antigen or hapten, comprising:
(a) contacting the fluid sample with a conjugate of a monomer and an antigen or hapten;
(b) contacting the sample of step (a) with a conjugate of a reporter and an antibody capable of specifically binding the antigen or hapten to form a reporter/antibody/antigen or hapten complex and a polymerizable reporter/antibody-monomer/antigen or hapten complex;
(c) separating the polymerizable reporter/antibody-monomer/antigen or hapten complex by initiating polymerization of the conjugate of the monomer and antigen or hapten.
(d) detecting the incorporation of reporter into the polymerized complex.

**18.** The immunoassay method of any of Claims 14 to 17, wherein the reporter is a radioisotope, an enzyme, an enzyme inhibitor, an enzyme cofactor, a fluorophore, a luminescent material, a chromophore and/or a particle.

**19.** The immunoassay method of any of Claims 14 to 17, wherein the monomer has the general formula:

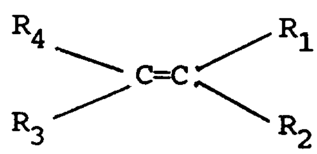

wherein $R_1$ is a hydrogen or lower alkyl having from 1 to 8 carbon atoms, $R_2$ is
-H
-COCl
-COOH
$-CO_2(CH_2)_n OH$ (n = 1-8)
$-CH_2NH_2$
$-CH_2Cl$
$-CO_2C_2H_4NHR$(R = H or any organic group)

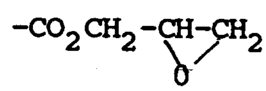

$-CO_2CH_2CHOHCH_2OH$
-CHO

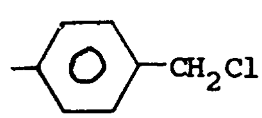

$CO_2(CH_2)_nNCO$($_n$ = 1-8)

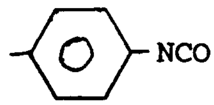

or

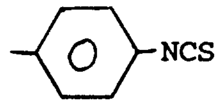

and $R_3$ and $R_4$ are H and/or residues which will provide a further unsaturated group.

**20.** The immunoassay method of Claim 19 wherein $R_3$ and $R_4$ are H.

**21.** The immunoassay method of any of claims 14 to 17, wherein the monomer comprises an acetylenically unsaturated monomer having a reactable group, which is capable of forming a covalent bond with an antibody, antigen or hapten or an intermediate spacer arm compound.

**22.** The immunoassay method of any of Claims 14 to 17, wherein the monomer is 2-hydroxyethyl methacrylate.

**23.** The immunoassay method of any of Claims 14 to 17, wherein the reporter is fluorescein isothiocyanate.

**Patentansprüche**

**1.** Ein Konjugat, das bei der de novo-Herstellung von Polymeren, die einen oder mehrere Antikörper enthalten, verwendet wird, umfassend: eine polymerisierbare Verbindung, enthaltend mindestens eine für die Bindung an einen Antikörper reaktive Stelle, und einen kovalent daran gebundenen Antikörper.

**2.** Das Konjugat nach Anspruch 1, worin der Antikörper ausgewählt wird aus der Gruppe, bestehend aus natürlich vorkommenden monoclonalen, polyclonalen, chemisch synthetisierten und mit Hilfe rekombinanter DNA (rDNA) hergestellter Antikörper.

**3.** Das Konjugat nach Anspruch 1, worin der Antikörper an eine Zwischenverbindung mit einem "Abstandshalter" gebunden ist, der ebenfalls an die polymerisierbare Verbindung gebunden ist.

**4.** Das Konjugat nach Anspruch 1, worin die polymerisierbare Verbindung in Wasser oder Mischungen aus Wasser/polarem organischen Lösungsmittel löslich ist.

**5.** Das Konjugat nach Anspruch 1 oder 3, das homopolymerisiert ist, um ein Polymer mit dem darin enthaltenen Antikörper zu bilden.

**6.** Das Konjugat nach Anspruch 1 oder 3, das mit einem nicht-derivatisierten Monomer copolymerisiert ist, um ein Copolymer mit dem darin enthaltenen Antikörper zu bilden.

**7.** Das Konjugat nach Anspruch 1 oder 3, worin die polymerisierbare Verbindung eine Verbindung ist, die olefinisch oder acetylenisch ungesättigte Reste und mindestens eine aktive Stelle für die Bindung mit dem Antikörper oder der Zwischenverbindung mit dem "Abstandshalter" enthält.

**8.** Das Konjugat nach Anspruch 1 oder 3, worin die polymerisierbare Verbindung ein mehrfach ungesättigtes Oligomer mit mindestens einer reaktiven Stelle für die Bindung mit dem Antikörper oder der Zwischenverbindung mit dem "Abstandshalter" ist.

**9.** Das Konjugat nach Anspruch 1, wobei die polymerisierbare Verbindung ein polymerisierbares ethylenisch ungesättigtes organisches Monomer umfaßt, ausgewählt aus der Gruppe, bestehend aus:

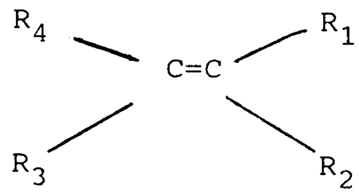

worin $R_1$ Wasserstoff oder einen niederen Alkylrest mit einem bis acht Kohlenstoffatomen darstellt, und $R_2$ wird ausgewählt aus der Gruppe, bestehend aus

-H
-COCl
-COOH
$-CO_2(CH_2)_nOH$ ($n$ = 1-8)
$-CH_2NH_2$
$-CH_2Cl$
$-CO_2C_2H_4NHR$ (R = H oder eine beliebige organische Gruppe)

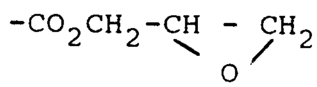

$-CO_2CH_2-CHOHCH_2OH$
-CHO

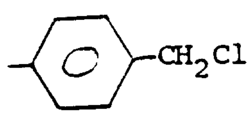

$-CO_2(CH_2)_nNCO$ (n = 1-8)

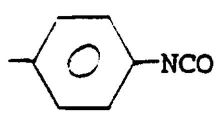

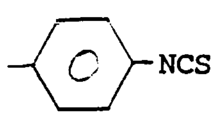

und $R_3$ und $R_4$ werden ausgewählt aus der Gruppe, bestehend aus H und/oder einem Rest, der eine weitere ungesättigte Gruppe bereitstellt.

**10.** Das Konjugat nach Anspruch 9, worin $R_3$ und $R_4$ Wasserstoff sind.

**11.** Ein Verfahren zum selektiven Entfernen eines interessierenden Antigens oder Haptens aus einer flüssigen Mischung, umfassend:

(a) Inkontaktbringen des Antigens oder Haptens in der flüssigen Mischung mit einem Konjugat aus einem Monomer und einem Antikörper, der eine spezifische Bindungsaktivität für das Antigen oder Hapten besitzt, um das Monomer an das Antigen oder Hapten zu binden, damit ein polymerisierbarer Monomer/Antikörper-Antigen- oder -Hapten-Komplex gebildet wird; und

(b) Polymerisieren des Komplexes, um ein unlösliches Polymer zu bilden, das von der flüssigen Mischung abgetrennt werden kann.

**12.** Das Verfahren nach Anspruch 11, worin die flüssige Mischung wäßrig ist.

**13.** Das Verfahren nach Anspruch 11, umfassend das Bereitstellen einer festen Oberfläche mit mindestens einer daran gebundenen polymerisierbaren Verbindung, die mit dem polymerisierbaren Konjugat aus einem Monomer und einem Antikörper copolymerisieren kann, und Polymerisieren des Konjugats in der Anwesenheit der an die feste Oberfläche gebundenen polymerisierbaren Verbindung.

**14.** Ein Immunassay-Verfahren zum Bestimmen der Anwesenheit eines Antigens oder Haptens in einer Flüssigkeitsprobe, von der angenommen wird, daß sie ein Antigen oder Hapten enthält, umfassend:

(a) Inkontaktbringen der das Antigen oder Hapten enthaltenden Flüssigkeitsprobe mit einem Konjugat aus einem Monomer und einem Antikörper, um einen polymerisierbaren Monomer/Antikörper-Antigen- oder -Hapten-Komplex auszubilden, und Bereitstellen eines Reportermoleküls zum Markieren des polymerisierbaren Monomer/Antikörper-Antigen- oder -Hapten-Komplexes;

(b) Abtrennen des markierten Komplexes durch Einleiten der Polymerisation des Komplexes; und

(c) Nachweisen des Einbaus des Reportermoleküls in den polymerisierten Komplex.

**15.** Das Immunassay-Verfahren nach Anspruch 14, worin der Reporter durch ein Reporter/Antikörper-Konjugat bereitgestellt wird, das das Antigen oder Hapten, das Konjugat aus einem Monomer und einem Antikörper oder den Monomer/Antikörper-Antigen- oder -Hapten-Komplex erkennen und binden kann.

**16.** Das Immunassay-Verfahren nach Anspruch 14, worin mehr als ein Antigen oder Hapten in der flüssigen Probe bestimmt wird und worin in Schritt (a) für jedes zu bestimmende Antigen oder Hapten ein Konjugat aus einem Monomer und einem Antikörper zugesetzt wird und worin weiterhin ein Reporter zum Markieren eines jeden der Monomer/Antikörper-Antigen- oder -Hapten-Komplexe bereitgestellt wird, wobei jeder Reporter ein zum Nachweisen von jedem in der flüssigen Probe vorhandenen anderen Reporter verschiedenes Signal bereitstellt, und worin in Schritt (c) der Einbau eines jeden Reporters in jedes der zuvor abgetrennten unlöslichen Polymere gemessen wird.

**17.** Ein Immunassay-Verfahren zum Bestimmen der Anwesenheit eines Antigens oder Haptens in einer Flüssigkeitsprobe, von der angenommen wird, daß sie das Antigen oder Hapten enthält, umfassend:

(a) Inkontaktbringen der Flüssigkeitsprobe mit einem Konjugat aus einem Monomer und einem Antigen oder Hapten;

(b) Inkontaktbringen der Probe aus Schritt (a) mit einem Konjugat aus einem Reportermolekül und einem Antikörper, der das Antigen oder Hapten spezifisch binden kann, um einen Reporter/Antikörper/Antigen- oder -Hapten-Komplex und einen polymerisierbaren Reporter/Antikörper-Monomer/ Antigen- oder -Hapten-Komplex zu bilden;

(c) Abtrennen des polymerisierbaren Reporter/AntikörperMonomer/Antigen- oder Hapten-Komplexes durch Einleiten der Polymerisation des Konjugats aus dem Monomer und Antigen oder Hapten;

(d) Nachweisen des Einbaus des Reporters in den polymerisierten Komplex.

**18.** Das Immunassay-Verfahren nach einem der Ansprüche 14 bis 17, worin der Reporter ein Radioisotop, ein Enzym, einen Enzyminhibitor, einen Enzymcofaktor, ein Fluorophor, ein Luminiszenzmaterial, ein Chromophor und/oder ein Partikel darstellt.

**19.** Das Immunassay-Verfahren nach einem der Ansprüche 14 bis 17, worin das Monomer die allgemeine Formel besitzt:

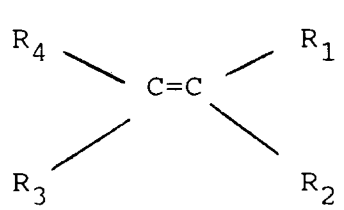

worin $R_1$ Wasserstoff oder einen niederen Alkylrest mit 1 bis 8 Kohlenstoffatomen darstellt, $R_2$ ist

-H
-COCl
-COOH
$-CO_2(CH_2)_nOH$ (n = 1-8)
$-CH_2NH_2$
$-CH_2Cl$
$-CO_2C_2H_4NHR$ (R = H oder eine beliebige organische Gruppe)

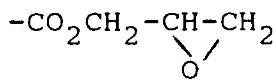

$-CO_2CH_2CHOHCH_2OH$
-CHO

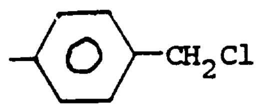

$-CO_2(CH_2)_nNCO$ (n = 1-8)

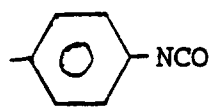

oder

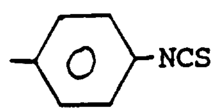

und $R_3$ und $R_4$ sind Wasserstoff und/oder Reste, die eine weitere ungesättigte Gruppe bereitstellen.

**20.** Das Immunassay-Verfahren nach Anspruch 19, worin $R_3$ und $R_4$ Wasserstoff darstellen.

**21.** Das Immunassay-Verfahren nach einem der Ansprüche 14 bis 17, worin das Monomer ein acetylenisch ungesättigtes Monomer mit einer reaktionsfähigen Gruppe umfaßt, die eine kovalente Bindung mit einem Antikörper, Antigen oder Hapten oder einer Zwischenverbindung mit Abstandshalter eingehen kann.

**22.** Das Immunassay-Verfahren nach einem der Ansprüche 14 bis 17, worin das Monomer 2-Hydroxyethylmethacrylat darstellt.

**23.** Das Immunassay-Verfahren nach einem der Ansprüche 14 bis 17, worin der Reporter Fluoresceinisothiocyanat darstellt.

## Revendications

**1.** Conjugué utilisé dans la nouvelle préparation de polymères contenant un ou plusieurs anticorps, comprenant :

un composé polymérisable contenant au moins un site réactif pour la liaison avec un anticorps, et un anticorps qui y est lié de façon covalente.

**2.** Conjugué selon la revendication 1, dans lequel l'anticorps est choisi parmi le groupe constitué des anticorps naturels monoclonaux, polyclonaux, synthétisés par voie chimique et dérivés d'ADN recombinant (ADNr).

**3.** Conjugué selon la revendication 1, dans lequel l'anticorps est lié à un composé intermédiaire de "bras espaceur" qui est aussi lié au composé polymérisable.

**4.** Conjugué selon la revendication 1, dans lequel le composé polymérisable est soluble dans l'eau ou dans des mélanges eau/solvant organique polaire.

**5.** Conjugué selon la revendication 1 ou 3, homopolymérisé pour former un polymère contenant intégralement l'anticorps.

**6.** Conjugué selon la revendication 1 ou 3, copolymérisé avec un monomère non dérivé pour former un copolymère contenant intégralement l'anticorps.

**7.** Conjugué selon la revendication 1 ou 3, dans lequel le composé polymérisable est un composé contenant une insaturation oléfinique ou acétylénique et au moins un site réactif pour la liaison avec l'anticorps ou le composé intermédiaire de "bras espaceur".

**8.** Conjugué selon la revendication 1 ou 3, dans lequel le composé polymérisable est un oligomère polyinsaturé ayant au moins un site réactif pour la liaison avec l'anticorps ou le composé intermédiaire de "bras espaceur".

**9.** Conjugué selon la revendication 1, le composé polymérisable comprenant un monomère organique polymérisable, éthyléniquement insaturé, choisi parmi le groupe constitué de :

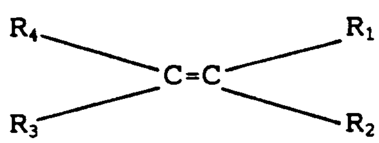

où $R_1$ est de l'hydrogène ou un radical alkyle inférieur ayant de un à huit atomes de carbone et $R_2$ est choisi parmi le groupe constitué de

-H
-COCl
-COOH
$-CO_2(CH_2)_nOH$ ($_n$ = 1 à 8)
$-CH_2NH_2$
$-CH_2Cl$
$-CO_2C_2H_4NHR$ (R = H ou tout groupe organique)

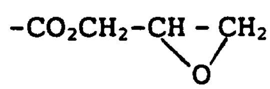

$-CO_2CH_2-CHOHCH_2OH$
-CHO

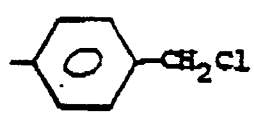

$-CO_2(CH_2)_nNCO$ ($_n$ = 1 à 8)

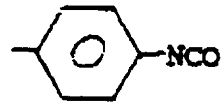

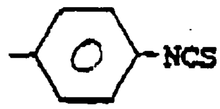

et $R_3$ et $R_4$ sont choisis parmi le groupe constitué de H et/ou d'un résidu qui fournit un groupe encore insaturé.

**10.** Conjugué selon la revendication 9, dans lequel $R_3$ et $R_4$ sont H.

**11.** Procédé d'élimination sélective d'un antigène ou d'un haptène intéressants d'un mélange fluide, comprenant :

a) la mise en contact de l'antigène ou du haptène dans le mélange fluide avec un conjugué d'un monomère et d'un anticorps qui a l'activité de liaison spécifique pour ledit antigène ou haptène, pour lier le monomère à l'antigène ou au haptène pour former un complexe monomère/anticorps-antigène ou haptène polymérisable ; et

b) la polymérisation du complexe pour former un polymère insoluble qui peut être séparé du mélange fluide.

**12.** Procédé selon la revendication 11, dans lequel le mélange fluide est aqueux.

**13.** Procédé selon la revendication 11, incluant l'apport d'une surface solide à laquelle au moins un composé polymérisable est lié, capable de se copolymériser avec le conjugué polymérisable d'un monomère et d'un anticorps, et la polymérisation du conjugué en présence du composé polymérisable lié à la surface solide.

**14.** Procédé d'immuno-essai pour déterminer la présence d'un antigène ou d'un haptène dans un échantillon fluide soupçonné de contenir un antigène ou un haptène, comprenant :

(a) la mise en contact de l'échantillon fluide contenant l'antigène ou le haptène avec un conjugué d'un monomère et d'un anticorps afin de former un complexe monomère/anticorps-antigène ou haptène polymérisable, et l'apport d'un reporteur pour marquer le complexe monomère/anticorps-antigène ou haptène polymérisable ;

(b) la séparation du complexe marqué en initiant la polymérisation du complexe ; et

(c) la détection de l'incorporation du reporteur dans le complexe polymérisé.

**15.** Procédé d'immuno-essai selon la revendication 14, dans lequel le reporteur est fourni par un conjugué reporteur/anticorps capable de reconnaître et de se lier à l'antigène ou au haptène, au conjugué d'un monomère et d'un anticorps ou au complexe monomère/anticorps-antigène ou haptène.

**16.** Procédé d'immuno-essai selon la revendication 14, dans lequel plus d'un antigène ou d'un haptène est déterminé dans l'échantillon fluide

et dans lequel, dans l'étape a), pour chaque antigène ou haptène devant être déterminé, on ajoute un conjugué d'un monomère et d'un anticorps, et ensuite un reporteur pour marquer chacun des complexes monomère/anticorps-antigène ou haptène est fourni, chaque reporteur fournissant un signal détecté différent de celui de chaque autre reporteur présent dans ledit échantillon fluide, et

dans lequel dans l'étape c) l'incorporation de chaque reporteur dans chacun des polymères insolubles séparés au préalable est détectée.

**17.** Procédé d'immuno-essai pour déterminer la présence d'un antigène ou d'un haptène dans un échantillon fluide soupçonné de contenir ledit antigène ou ledit haptène, comprenant :

(a) la mise en contact de l'échantillon fluide avec un conjugué d'un monomère et d'un antigène ou haptène ;

b) la mise en contact de l'échantillon de l'étape a) avec un conjugué d'un reporteur et d'un anticorps capable de se lier spécifiquement à l'antigène ou au haptène pour former un complexe reporteur/anticorps/antigène ou haptène et un complexe reporteur/anticorpsmonomère/antigène ou haptène polymérisable ;
c) la séparation du complexe reporteur/anticorpsmonomère/antigène ou haptène polymérisable en initiant la polymérisation du conjugué du monomère et de l'antigène ou haptène ;
d) la détection de l'incorporation du reporteur dans le complexe polymérisé.

**18.** Procédé d'immuno-essai selon l'une quelconque des revendications 14 à 17, dans lequel le reporteur est un radio-isotope, une enzyme, un inhibiteur d'enzyme, un cofacteur d'enzyme, un fluorophore, une matière luminescente, un chromophore et/ou une particule.

**19.** Procédé d'immuno-essai selon l'une quelconque des revendications 14 à 17, dans lequel le monomère est de formule générale :

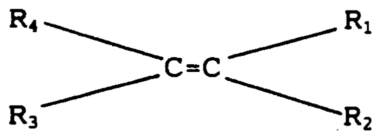

où $R_1$ est un hydrogène ou un radical alkyle inférieur ayant de 1 à 8 atomes de carbone, $R_2$ est
-H
-COCl
-COOH
$-CO_2(CH_2)_nOH$ ($_n$ = 1 à 8)
$-CH_2NH_2$
$-CH_2Cl$
$-CO_2C_2H,NHR$ (R = H ou tout groupe organique)

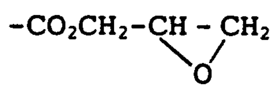

$-CO_2CH_2-CHOHCH_2OH$
-CHO

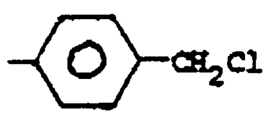

$-CO_2(CH_2)_nNCO$ ($_n$ = 1 à 8)

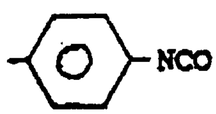

ou

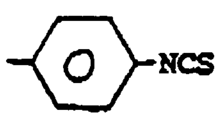

et $R_3$ et $R_4$ sont H et/ou des résidus qui fourniront un groupe encore insaturé.

**20.** Procédé d'immuno-essai selon la revendication 19, dans lequel $R_3$ et $R_4$ sont H.

**21.** Procédé d'immuno-essai selon l'une quelconque des revendications 14 à 17, dans lequel le monomère comprend un monomère acétyléniquement insaturé ayant un groupe pouvant réagir, qui est capable de former une liaison covalente avec un anticorps, un antigène ou un haptène ou un composé intermédiaire de bras espaceur.

**22.** Procédé d'immuno-essai selon l'une quelconque des revendications 14 à 17, dans lequel le monomère est du méthacrylate de 2-hydroxyéthyle.

**23.** Procédé d'immuno-essai selon l'une quelconque des revendications 14 à 17, dans lequel le reporteur est de l'isothiocyanate de fluorescéine.

A.

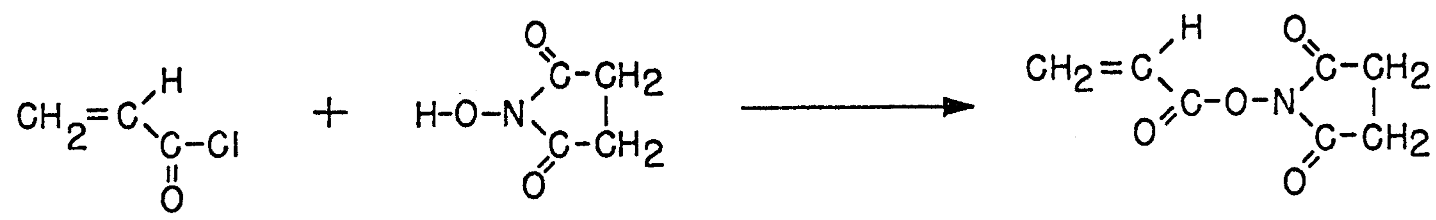

ACRYLOYL CHLORIDE

N-HYDROXY-SUCCINIMIDE

N-HYDROXYSUCCINIMIDE ESTER OF ACRYLIC ACID

B.

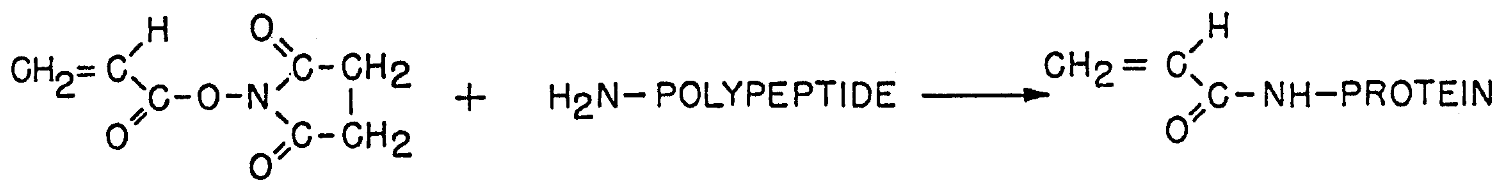

N-HYDROXYSUCCINIMIDE ESTER OF ACRYLIC ACID

POLYPEPTIDE WITH AMINO GROUPS

MONOMER / POLYPEPTIDE CONJUGATE

FIG. 1

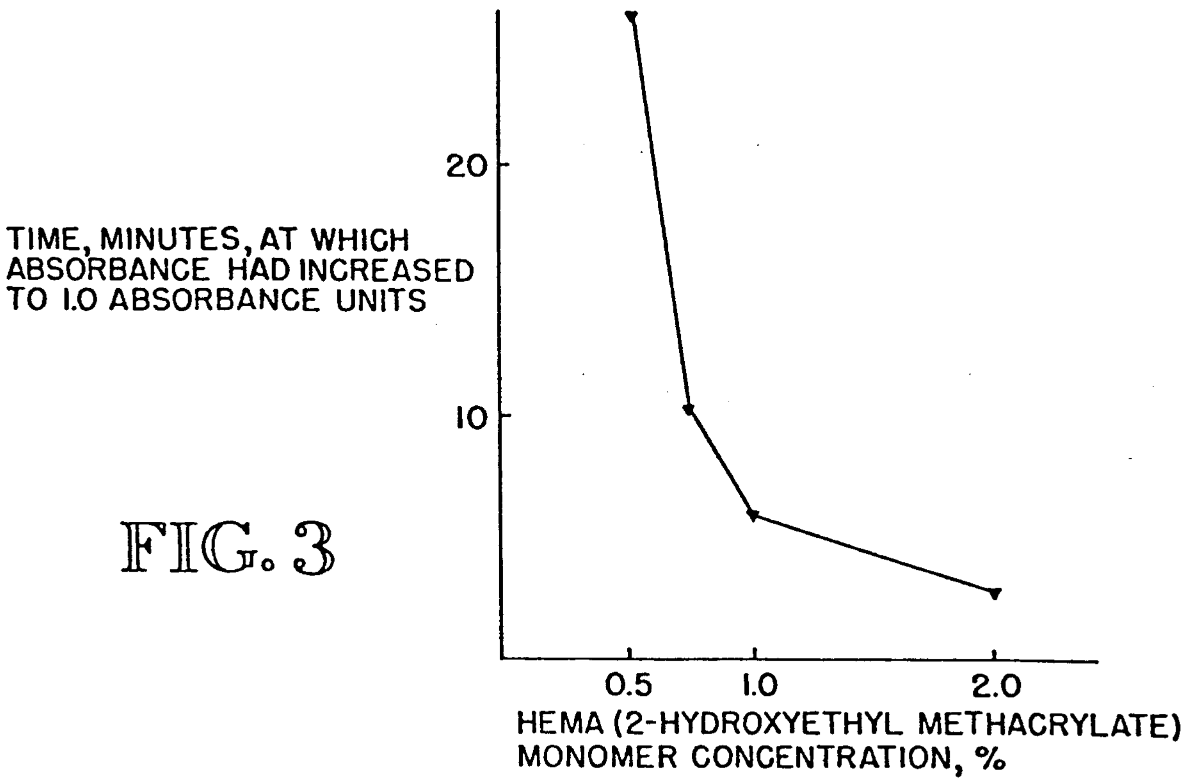
20
10
TIME, MINUTES, AT WHICH ABSORBANCE HAD INCREASED TO 1.0 ABSORBANCE UNITS
0.5
1.0
2.0
HEMA (2-HYDROXYETHYL METHACRYLATE) MONOMER CONCENTRATION, %

FIG. 3

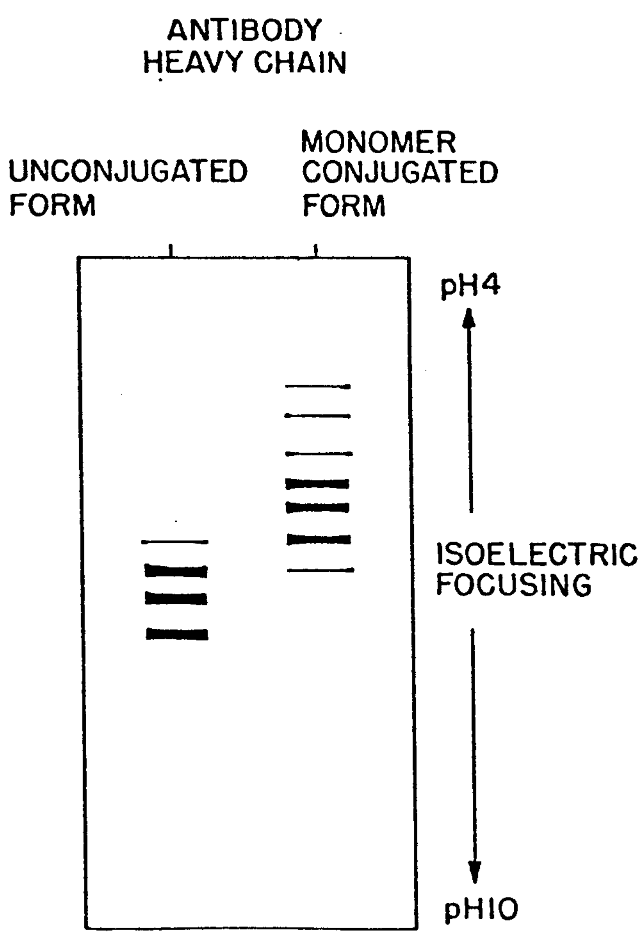
ANTIBODY HEAVY CHAIN
UNCONJUGATED FORM
MONOMER CONJUGATED FORM
pH4
ISOELECTRIC FOCUSING
pH10

FIG. 2

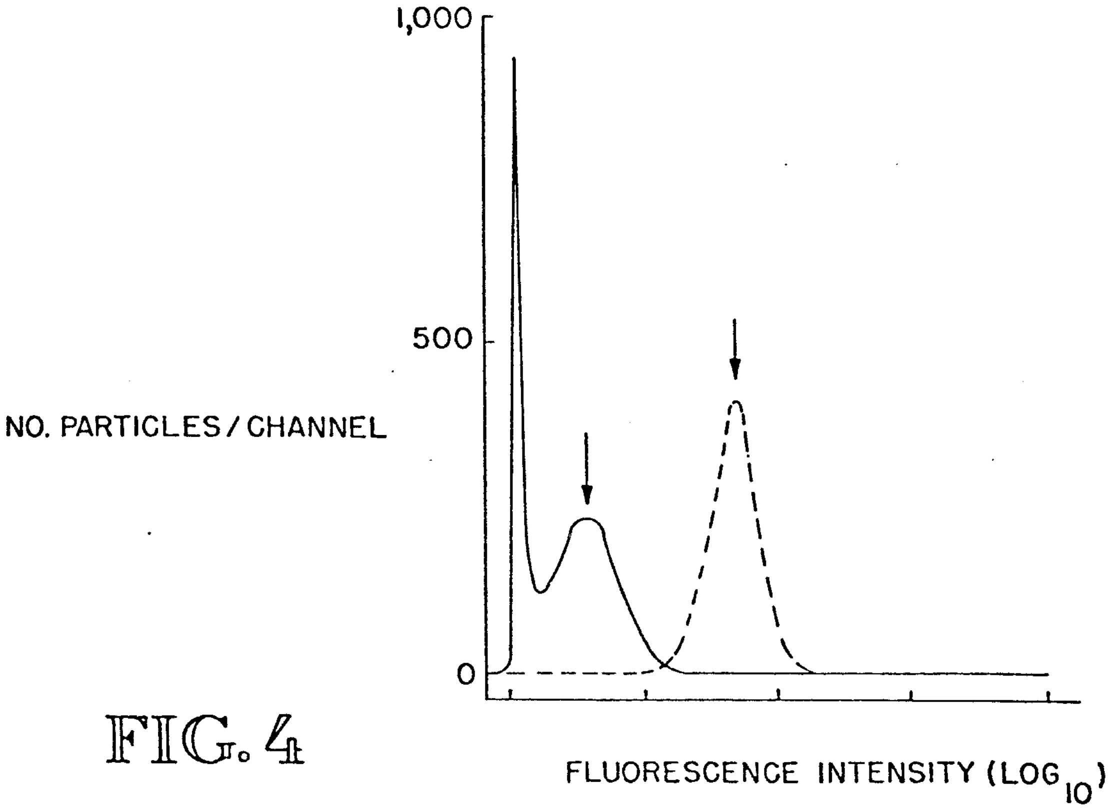
1,000
500
0
NO. PARTICLES / CHANNEL
FLUORESCENCE INTENSITY (LOG 10)

FIG. 4

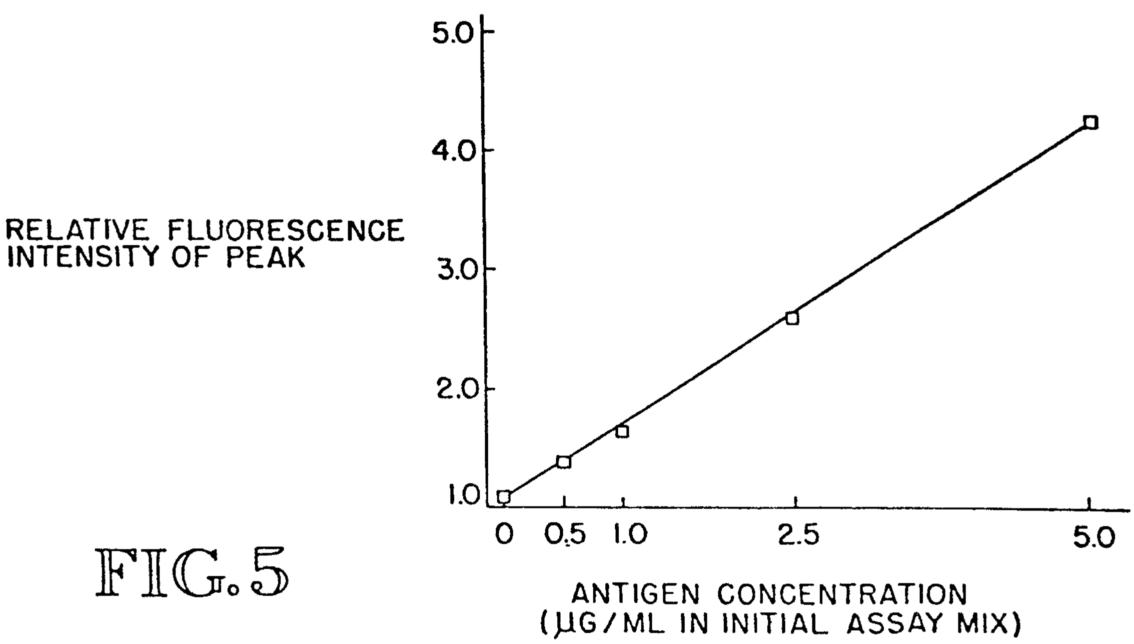
5.0
4.0
3.0
2.0
1.0
RELATIVE FLUORESCENCE INTENSITY OF PEAK
0
0.5
1.0
2.5
5.0
ANTIGEN CONCENTRATION
(µG / ML IN INITIAL ASSAY MIX)

FIG. 5

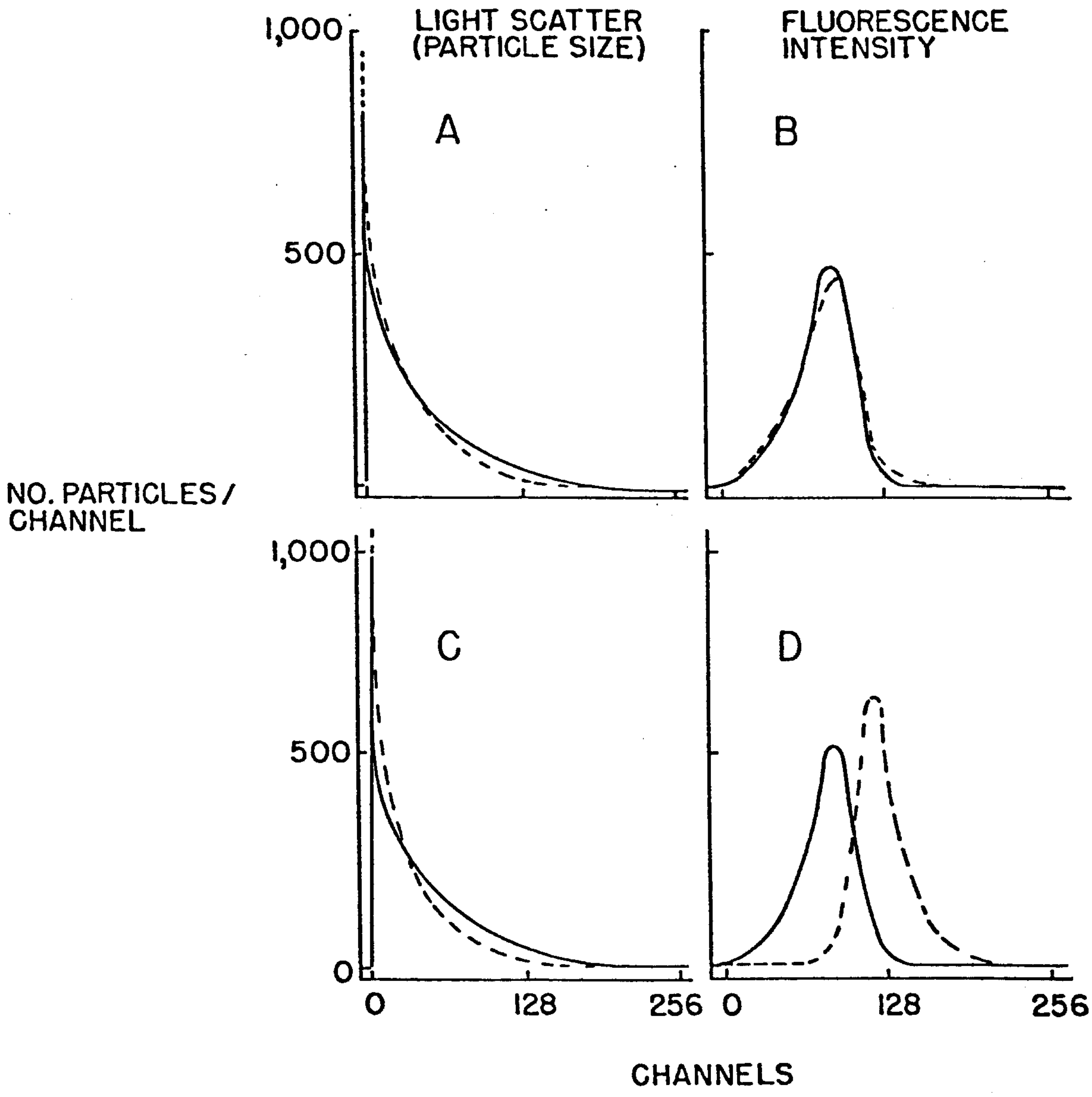
LIGHT SCATTER
(PARTICLE SIZE)
FLUORESCENCE
INTENSITY
A
B
C
D
NO. PARTICLES / CHANNEL
1,000
500
0
0
128
256
CHANNELS

FIG. 6